# EUROPÄISCHE PATENTSCHRIFT

(11) **EP 3 134 380 B1**
(45) Veröffentlichungstag und Bekanntmachung des Hinweises auf die Patenterteilung: **14.08.2019**
(21) Anmeldenummer: 15720628.5
(22) Anmeldetag: 20.04.2015
(51) Int. Cl.: C07C 67/08, C08K 5/00, C08K 5/10

(54) **VERFAHREN ZUR HERSTELLUNG VON CARBONSÄUREESTERN IN GEGENWART EINER IONISCHEN FLÜSSIGKEIT UND EINES SAUREN VERESTERUNGSKATALYSATORS UND DEREN VERWENDUNG ALS WEICHMACHER**
METHOD FOR PRODUCING CARBOXYLIC ACID ESTERS IN THE PRESENCE OF AN IONIC LIQUID AND AN ACIDIC ESTERIFICATION CATALYST AND THE USE THEREOF AS PLASTICIZER
PROCÉDÉ DE PRODUCTION D'ESTERS D'ACIDES CARBOXYLIQUES EN PRÉSENCE D'UN LIQUIDE IONIQUE ET D'UN CATALYSEUR D'ESTÉRIFICATION ACIDE ET LEUR UTILISATION COMME PLASTIFIANT

(30) Priorität: 22.04.2014 EP 14165458
(43) Veröffentlichungstag der Anmeldung: 01.03.2017
(73) Patentinhaber: BASF SE, 67056 Ludwigshafen am Rhein (DE)
(72) Erfinder: KALLER, Martin, 68163 Mannheim (DE); KOCH, Michael, 67346 Speyer (DE)
(74) Vertreter: BASF IP Association
(86) Internationale Anmeldenummer: PCT/EP2015/058482
(87) Internationale Veröffentlichungsnummer: WO 2015/162079

(56) Entgegenhaltungen:
- WO-A1-2010/076192
- CN-A- 102 001 948
- US-A- 1 923 938
- US-A- 2 628 207
- Cadogan, Howick: "Plasticizers" In: "Ullmann's Encyclopedia of Industrial Chemistry", 2012, Wiley-VCH Verlag GmbH & Co. KGaA, Weinheim, Germany, XP055167665, ISBN: 978-3-52-730673-2 Seiten 599-618, DOI: 10.1002/14356007.a20_439, Das ganze Dokument, z.B. Seite 600, Tabelle 1.
- Lorz et al.: "Phthalic Acid and Derivatives" In: "Ullmann's Encyclopedia of Industrial Chemistry", 2012, Wiley-VCH Verlag GmbH & Co. KGaA, Weinheim, Germany, XP055167648, ISBN: 978-3-52-730673-2 Seiten 131-180, DOI: 10.1002/14356007.a20_181.pub2, in der Anmeldung erwähnt Seiten 143-148, Kapitel 5 bis 5.5.
- OXEA GmbH: "Oxsoft / Oxblue Brochure", , Februar 2014 (2014-02), Seiten 1-20, XP055202678, Gefunden im Internet: URL:http://ox-rch.by.nf/fileadmin/phthalat e/OXSOFT/OXSOFT_OXBLUE-Bruchure.pdf [gefunden am 2015-07-16]
- ZHU ET AL.: "Brønsted acidic ionic liquid 1-methylimidazolium tetrafluoroborate: a green catalyst and recyclable medium for esterification", GREEN CHEMISTRY, Bd. 5, Nr. 1, 1. Januar 2003 (2003-01-01), Seiten 38-39, XP55203035, ISSN: 1463-9262, DOI: 10.1039/b209248b
- NGUYEN ET AL.: "An improved greener esterification of fatty alcohols using a renewable acid-ionic liquid couple as catalyst-solvent", SYNTHETIC COMMUNICATIONS, Bd. 34, Nr. 11, 1. Januar 2004 (2004-01-01), Seiten 2085-2093, XP008086678, ISSN: 0039-7911, DOI: 10.1081/SCC-120037923
- CAI ET AL.: "Imidazolium ionic liquid-supported sulfonic acids: Efficient and recyclable catalysts for esterification of benzoic acid", CHINESE CHEMICAL LETTERS, Bd. 23, Nr. 1, 2012, Seiten 1-4, XP028393378, ISSN: 1001-8417, DOI: 10.1016/J.CCLET.2011.09.016 [gefunden am 2011-09-21]
- XIE ET AL.: "Synthesis of plasticizer ester using acid-functionalized ionic liquid as catalyst", JOURNAL OF HAZARDOUS MATERIALS, Bd. 151, Nr. 2-3, 4. Dezember 2007 (2007-12-04), Seiten 847-850, XP022450980, ISSN: 0304-3894, DOI: 10.1016/J.JHAZMAT.2007.11.113
- "Fluka-Riedel-de Haën Katalog: Laborchemikalien und analytische Reagentien 2005/2006", 1. Januar 2005 (2005-01-01), Fluka, Buchs (CH), XP055203357, Seite 756, 757, 1005, 1006, das ganze Dokument

## Beschreibung

### HINTERGRUND DER ERFINDUNG

Die vorliegende Erfindung betrifft ein Verfahren zur Herstellung von Carbonsäureestern durch Umsetzung von Carbonsäuren und/oder Carbonsäureanhydriden mit wenigstens einem Alkohol mit mindestens 5 Kohlenstoffatomen, ausgewählt unter Alkanolen und Cycloalkanolen, in Gegenwart einer ionischen Flüssigkeit sowie eines sauren Veresterungskatalysators. Die Erfindung betrifft weiterhin die Verwendung der so erhaltenen Carbonsäureester als Weichmacher oder in einer Weichmacherzusammensetzung für thermoplastische Polymere und Elastomere.

### STAND DER TECHNIK

Ester aliphatischer und aromatischer Carbonsäuren mit Alkanolen und Cycloalkanolen mit mindestens 5 Kohlenstoffatomen haben in der Technik vielfältig Verwendung gefunden. Sie finden beispielsweise weite Anwendung in Lackharzen und als Bestandteile von Anstrichmitteln, wobei speziell die Ester der Phthalsäure, Trimellitsäure, Terephthalsäure, Adipinsäure, Sebacinsäure oder Maleinsäure eingesetzt werden. Sie eignen sich des Weiteren speziell als Weichmacher oder als Komponente einer Weichmacherzusammensetzung für thermoplastische Polymere und Elastomere.

Weichmacher werden einer Vielzahl von Kunststoffen zur Erzielung der gewünschten Verarbeitungs- bzw. Anwendungseigenschaften zugesetzt, um diese weicher, flexibler und/oder dehnbarer zu machen. Im Allgemeinen dient der Einsatz von Weichmachern dazu, den thermoplastischen Bereich von Kunststoffen zu niedrigeren Temperaturen hin zu verschieben, um im Bereich niedriger Verarbeitungs- und Einsatztemperaturen die gewünschten elastischen Eigenschaften zu erhalten. Wichtige thermoplastische Polymere in denen üblicherweise Weichmacher Anwendung finden sind neben Polyvinylchlorid (PVC) beispielsweise Polyvinylbutyral (PVB), Homo- und Copolymere von Styrol, Polyacrylate, Polysulfide oder thermoplastische Polyurethane (PU). Aufgrund ihrer guten Verträglichkeit mit PVC und weiteren Polymeren und ihrer vorteilhaften anwendungstechnischen Eigenschaften wurden in der Vergangenheit vielfach Phthalsäurediester mit Alkoholen unterschiedlicher chemischer Struktur als Weichmacher eingesetzt, wie z. B. Diethylhexylphthalat (DEHP). Da diese jedoch toxikologisch nicht unbedenklich sind, wurden sie in letzter Zeit speziell für sensible Anwendungsbereiche wie Kinderspielzeug, Lebensmittelverpackungen oder medizinische Artikel durch andere Weichmacher ersetzt. Hier sind insbesondere die Ester weiterer aromatischer Carbonsäuren, wie der Terephthalsäure, Trimellitsäure und Benzoesäure von Bedeutung.

Es ist bekannt, Carbonsäureester durch Umsetzung von Carbonsäuren mit Alkoholen herzustellen. Diese Reaktion kann autokatalytisch oder katalytisch, beispielsweise durch Brönsted- oder Lewissäuren, durchgeführt werden. Derartige Verfahren sind in Lorz et al., Phthalic Acid and Derivatives, Ullmann's Encyclopedia of Industrial Chemistry, 2007, Seiten 131 - 180 (DOI: 10.1002/14356007.a20_181.pub2) beschrieben. Bei der autokatalytischen Veresterung liegen die Reaktionstemperaturen üblicherweise bei > 200 °C. Dennoch werden in der Regel nur Teilumsätze erreicht, so dass ein Recycling der zurückbleibenden Carbonsäure zwingend erforderlich ist.

Unabhängig von der Art der Katalyse entsteht immer ein temperaturabhängiges Gleichgewicht zwischen den Einsatzstoffen (Carbonsäure und Alkohol) und den Produkten (Ester und Wasser). Die Umsetzung von inneren Carbonsäureanhydriden mit Alkoholen verläuft in zwei Schritten: Die Alkoholyse des Anhydrids zum Monoester verläuft in der Regel rasch und vollständig. Die weitere Umsetzung des Monoesters zum Diester unter Bildung von Reaktionswasser ist reversibel und verläuft langsam. Dieser zweite Schritt ist der geschwindigkeitsbestimmende Schritt der Reaktion. Um das Gleichgewicht zu Gunsten des Esters (bzw. des Vollesters bei mehrbasigen Säuren) zu verschieben, wird in der Regel ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von höheren aliphatischen Carbonsäuren, aromatischen Carbonsäuren oder von zwei- oder mehrbasigen Carbonsäuren ist in der Regel der eingesetzte Alkohol das Schleppmittel.

Typische Veresterungskatalysatoren zur Herstellung von als Weichmachern geeigneten Carbonsäureestern sind Tetraalkyltitanate.

US 7,799,942 beispielsweise offenbart ein Verfahren zur Herstellung von Diestern der Terephthalsäure, wie beispielsweise Bis(2-ethylhexyl)terephthalat (DOTP), bei dem man Terephthalsäure und einen C₆-C₁₀-Alkohol einer Veresterung in Gegenwart eines Tetraalkyltitanats als Katalysator unterzieht, wobei das während der Veresterung entstehende Wasser und ein Teil des Alkohols durch Hindurchleiten eines Inertgases durch die Reaktionszone oder mit Hilfe einer Destillationskolonne entfernt werden.

Die WO 2010/076192 beschreibt ein Verfahren zur Herstellung von Carbonsäureestern durch Umsetzung einer Carbonsäure und/oder eines Carbonsäureanhydrids mit einem Alkohol in Gegenwart, bei dem man das entstehende Reaktionswasser als Alkohol-Wasser-Azeotrop abdestilliert, zumindest teilweise kondensiert, das Kondensat in eine wässrige und organische Phase auftrennt und die organische Phase nach Entfernung niedriger als der Alkohol siedender Komponenten ("Leichtsiederabtrennung") wieder in das Reaktionssystem zurückführt. Als Veresterungskatalysatoren werden MetallAlkoholate und insbesondere Tetraalkyltitanate eingesetzt.

Der Einsatz von Tetraalkyltitanaten als Katalysatoren ist mit mehreren Nachteilen verbunden. So wird das Reaktionsgemisch zur Entfernung des Katalysators mit einer Base, z. B. wässriger NaOH, versetzt und die resultierenden Hydrolyseprodukte werden abfiltriert. Diese Abtrennung ist zeitaufwändig, so dass nur geringe Raum-Zeit-Ausbeuten erzielt werden. In der Regel ist eine weitere Aufarbeitung des Reaktionsgemisches erforderlich, z. B. eine Destillation zur Entfernung überschüssigen Alkohols und/oder eine Behandlung mit Aktivkohle zur Erzielung akzeptabler Farbzahlen.

Des Weiteren ist im Stand der Technik auch der Einsatz von Mineralsäuren und starken organischen Säuren, wie Methansulfonsäure und p-Toluolsulfonsäure, als Katalysatoren zur Herstellung von Carbonsäureestern beschrieben. In Lorz et al., Phthalic Acid and Derivatives, Ullmann's Encyclopedia of Industrial Chemistry, 2007, Seiten 131 - 180 (DOI: 10.1002/14356007.a20_181.pub2) wird jedoch gelehrt, dass Brönstedsaure Katalysatoren, wie Schwefelsäure oder Sulfonsäuren, nur bis zu einer Temperatur von 165 °C eingesetzt werden können, da ansonsten störende Nebenreaktionen auftreten, die zur Bildung von Ethern, Sulfonsäureestern, Olefinen (durch Wassereliminierung aus den eingesetzten Alkoholen) oder zu anderen Nebenprodukten führen können, welche die Veresterungsprodukte in unerwünschter Weise verfärben.

Die JP 62267341 offenbart ein Verfahren zur Herstellung von Carbonsäureestern zur Verwendung als Weichmacher, erhältlich durch Umsetzung einer Carbonsäure mit einem Alkohol in Gegenwart einer Sulfonsäure als Veresterungskatalysator. Das Veresterungsrohprodukt wird dabei einer Reinigung durch Zugabe einer Base, z. B. CaO oder MgO, und eines festen Adsorbens, wie beispielsweise Aktivkohle, Kieselgur oder aktivierte Bleicherde, zur Verringerung der Säurezahl bzw. der Farbzahl unterzogen.

Die WO 2008/123928 beschreibt ein Verfahren zur Herstellung von Di-(n-butyl)-terephthalat aus Terephthalsäure und n-Butanol, wobei die Veresterung mit einem 1,25- bis 4-fachen molaren Überschuss an n-Butanol bei Normaldruck und einer Reaktionstemperatur zwischen 110 und 220 °C unter Verwendung eines Veresterungskatalysators durchgeführt wird. Konkret wird dieses Verfahren aufgrund der Siedetemperatur des n-Butanols von 117 °C bevorzugt bei einer Reaktionstemperatur von 115 bis 150 °C (d. h. im Wesentlichen unter Rückfluss) durchgeführt, wobei als Veresterungskatalysator bevorzugt eine Sulfonsäure oder Schwefelsäure eingesetzt wird. Während der Reaktion wird der Reaktionszone kontinuierlich n-Butanol zugeführt. Das während der Reaktion entstehende Wasser wird destillativ als azeotropes Gemisch abgeführt. In einigen der Ausführungsbeispiele wird Stickstoff durch die Reaktionsmischung geleitet, wobei diese Maßnahme jedoch keinen erkennbaren positiven Einfluss auf die Ausbeute, Reinheit oder Farbzahl des erhaltenen Veresterungsproduktes hat.

In der Regel müssen die in diesen Verfahren als Katalysator eingesetzten Brönsted-Säuren, wie Sulfonsäuren oder Schwefelsäure, am Ende der Reaktion in einer basischen Wäsche entfernt werden, wodurch diese nicht wiederverwendet werden können.

Veresterungsverfahren, bei denen ionische Flüssigkeiten zum Einsatz kommen sind ebenfalls im Stand der Technik bekannt.

CN102001948A beschreibt ein Verfahren zur Herstellung von Diisooctylterephthalat (DOTP) aus Terephthalsäure und 2-Ethylhexanol, bei dem als Veresterungskatalysator eine ionische Flüssigkeit, erhältlich aus der Reaktion von 1-Butylpyridiniumchlorid mit Zinn(II)-chlorid, verwendet wird.

Xie et al., Journal of Hazardous Materials, 2007, 151, 847-850, beschreiben ein Verfahren zur Herstellung von Estern, wie Dioctylphthalat (DOP) oder Dioctylsebacat (DOS), bei dem Sulfonsäuregruppen-modifizierte ionischer Flüssigkeiten als Veresterungskatalysatoren eingesetzt werden, wobei das bei der Veresterung entstehende Wasser unter Verwendung eines Wasserabscheiders aus der Reaktionsmischung entfernt wird. Bei den ionischen Flüssigkeiten handelt es sich insbesondere um Sulfonsäuregruppen-modifizierte Imidazolium- und Pyridiniumsalze. Einige dieser ionische Flüssigkeiten können nach Entfernung von Wasser wiederverwendet werden.

CN102824929A beschreibt ein Verfahren zur Herstellung von Dioctylterephthalat (DOTP) aus Terephthalsäure und 2-Ethylhexanol, bei dem ein Katalysatorsystem zum Einsatz kommt, welches aus zwei Katalysatoren, Katalysator 1 und Katalysator 2, besteht. Katalysator 1 ist eine Mischung aus einer ionischen Flüssigkeit und einer Metallverbindung. Die ionische Flüssigkeit ist dabei ausgewählt unter Imidazolium- oder Benzimidazolium-Ionen, die ein Sulfat-, Bisulfat- oder ein Nitration als Gegenionen tragen und gegebenenfalls mit Sulfonsäuregruppen substituiert sein können. Katalysator 2 ist eine Mischung aus einer Titan-Metallverbindung und einer weiteren Metallverbindung. Konkret wird bei dem Verfahren zu einer Mischung der beiden Einsatzstoffe der Katalysator 1 bei einer Reaktionstemperatur von 130 bis 150 °C zugegeben, gefolgt von der Zugabe des Katalysators 2 nach einer weiteren Temperaturerhöhung auf 160 bis 220°C, wobei die Reaktion noch für weitere 0,5 bis 3 Stunden unter Rückfluss gerührt und das während der Reaktion entstehende Wasser destillativ als azeotropes Gemisch abgeführt wird.

CN102329233A beschreibt ein Verfahren zur Synthese von Diisooctylterephthalat (DOTP) bei dem Terephthalsäure und 2-Ethylhexanol in Gegenwart von Tetrabutyltitanat als primärer Veresterungskatalysator und einer ionischen Flüssigkeit, bestehend aus einem quaternären Ammoniumsalz, als Co-Katalysator und Lösungsvermittler, in einer Veresterungsreaktion umgesetzt wird.

In vielen Fällen sind die zur Herstellung von Weichmachern eingesetzten Carbonsäuren, wie beispielsweise die Terephthalsäure, in den zur Veresterung verwendeten Alkoholen, wie beispielsweise 2-Ethylhexanol, nur schlecht löslich. Dadurch erhöht sich die Gefahr der Nebenproduktbildung bei den herkömmlichen Titan- bzw. säurekatalysierten Veresterungsverfahren, da lange Reaktionszeiten und/oder erhöhte Reaktionstemperaturen benötigt werden, um hohe Umsätze zu erzielen.

Der vorliegenden Erfindung liegt die Aufgabe zugrunde, ein verbessertes Verfahren zur Herstellung von Carbonsäureestern zur Verfügung zu stellen, die sich für einen Einsatz als Weichmacher eignen. Dabei soll vorzugsweise ein möglichst vollständiger Umsatz nach kurzer Reaktionszeit und damit eine hohe Raum-Zeit-Ausbeute erzielt werden, wobei gleichzeitig die Bildung von Nebenprodukten minimiert werden soll. Des Weiteren soll das Verfahren kostengünstig und technisch einfach durchzuführen sein, z. B. durch die Verwendung eines preiswerten Katalysators und der Vermeidung aufwändiger Aufarbeitungsschritte, womit die oben beschriebenen Nachteile, die sich u. A. bei der Verwendung von Metallverbindungen, wie Tetraalkyltitanaten, als Veresterungskatalysatoren ergeben, weitgehend vermieden werden können. Das Verfahren soll zudem eine Wiederverwertung des Katalysators ermöglichen. Die erhaltenen Carbonsäureester sollen sich trotzdem durch gute Produkteigenschaften, speziell für eine Anwendung als Weichmacher, auszeichnen. Dazu zählt für Anwendungen in Bereichen, bei denen die optischen Eigenschaften der weichgemachten Kunststoffe von Bedeutung sind, eine möglichst geringe Färbung der Carbonsäureester, die sich beispielsweise an einer niedrigen Farbzahl zeigt.

Überraschenderweise wurde nun gefunden, dass diese Aufgabe gelöst wird, wenn man die Veresterung zur Herstellung der als Weichmacher geeigneten Carbonsäureester bei hohen Temperaturen in Gegenwart einer organischen Sulfonsäure, speziell Methansulfonsäure, als Katalysator und in Gegenwart einer ionischen Flüssigkeit durchführt, wobei der zur Veresterung eingesetzte Alkohol als Schleppmittel für das gebildete Reaktionswasser dient und nach Wasserabtrennung in die Reaktion zurückgeführt wird. In einer speziellen Ausführung wird als Katalysator Methansulfonsäure mit einem geringen Gesamtchlor- und Sulfatgehalt eingesetzt.

### ZUSAMMENFASSUNG DER ERFINDUNG

Gegenstand der Erfindung ist ein Verfahren zur Herstellung von Carbonsäureestern, durch Umsetzung wenigstens einer Carbonsäure und/oder wenigstens eines Carbonsäureanhydrids, ausgewählt unter aromatischen Mono-, Di-, Tri- oder Tetracarbonsäuren, aliphatischen Mono- und Dicarbonsäuren, Hydroxycarbonsäuren, alicyclischen Mono-, Di-, Tri- und Tetracarbonsäuren, heterocyclischen Dicarbonsäuren, den Anhydriden der zuvor genannten Carbonsäuren und Mischungen davon, und wenigstens eines Alkohols R¹-OH, worin R¹ ausgewählt ist unter unverzweigten und verzweigten gesättigten C₅-C₁₃-Alkylresten, mit der Maßgabe, dass die Umsetzung
- in Gegenwart wenigstens einer ionischen Flüssigkeit, die ausgewählt ist unter Salzen der allgemeinen Formel (la), 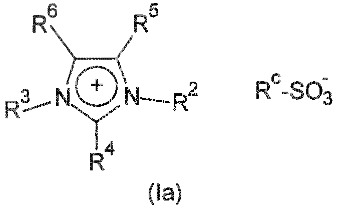 worin Re
   ste R² und R³ unabhängig voneinander für Wasserstoff, unsubstituiertes C₁-C₆-Alkyl, unsubstituiertes C₂-C₆-Alkenyl, unsubstituiertes C₅-C₆-Cycloalkyl, C₆-C₁₀-Aryl oder Heteroaryl mit 5 bis 6 Ringatomen, stehen, wobei die 2 letztgenannten Reste auch 1, 2 oder 3 Substituenten, ausgewählt unter C₁-C₆-Alkyl, C₁-C₆-Alkoxy oder Halogen, aufweisen können,
   Reste R⁴, R⁵, und R⁶ unabhängig voneinander für Wasserstoff, unsubstituiertes C₁-C₆-Alkyl, unsubstituiertes C₅-C₆-Cycloalkyl oder Benzyl stehen, wobei Benzyl auch 1, 2 oder 3 Substituenten, ausgewählt unter C₁-C₆-Alkyl, C₁-C₆-Alkoxy oder Halogen, aufweisen kann,
      und
   R^{c} für unsubstituiertes C₁-C₆-Alkyl, unsubstituiertes C₅-C₆-Cycloalkyl oder Aryl steht, wobei Aryl mit 1, 2, oder 3 C₁-C₆-Alkylresten substituiert sein kann,
- in Gegenwart wenigstens eines Katalysators, der ausgewählt ist unter organischen Sulfonsäuren und
- unter destillativer Abtrennung wenigstens eines Teils des während der Reaktion gebildeten Wassers in Form einer azeotropen Mischung mit dem eingesetzten Alkohol R¹-OH,
erfolgt, wobei der abdestillierte Alkohol R¹-OH zumindest teilweise in das Reaktionssystem zurückgeführt wird.

In einer speziellen Ausführungsform dient das erfindungsgemäße Verfahren zur Herstellung von Estern der Terephthalsäure, ganz speziell zur Herstellung von Bis(2-ethylhexyl)terephthalat (DOTP).

Ein weiterer Gegenstand der Erfindung ist die Verwendung der so erhaltenen Carbonsäureester als Weichmacher oder als Komponente in einer Weichmacherzusammensetzung für thermoplastische Polymere und Elastomere.

### BESCHREIBUNG DER ERFINDUNG

Das erfindungsgemäße Verfahren hat folgende Vorteile:
- Es wird die Herstellung von als Weichmacher geeigneten Carbonsäureestern bei kurzen Reaktionszeiten, d. h. mit einer hohen Raum-Zeit-Ausbeute ermöglicht.
- Die Carbonsäureester werden trotz der relativ drastischen Reaktionsbedingungen in hohen Ausbeuten und guten Selektivitäten erhalten.
- Obwohl eine Brönstedsäure als Katalysator eingesetzt wird, wird die Bildung unerwünschter Nebenprodukte, speziell von Sulfonsäureestern oder Ethern des zur Veresterung eingesetzten Alkohols und von Olefinen aus der Wassereliminierung des Alkohols, nur in äußerst geringem Umfang beobachtet.
- Die in dem erfindungsgemäßen Veresterungsverfahren eingesetzte ionische Flüssigkeit wirkt in vorteilhafter Weise als Lösungsvermittler zwischen der Carbonsäure und dem Alkohol, wodurch die Reaktionszeit verringert werden kann.
- Die als Katalysator eingesetzte Brönstedsäure kann zusammen mit der ionischen Flüssigkeit vom Reaktionsgemisch abgetrennt und für weitere Veresterungsreaktionen wiederverwendet werden.
- Auf den Einsatz aufwändiger Maßnahmen zur Reinigung der nach dem erfindungsgemäßen Verfahren erhaltenen Carbonsäureester kann in der Regel verzichtet werden. Dies gilt speziell für den Einsatz von Adsorbentien zur Erzielung weniger gefärbter Produkte.
- Auf den Einsatz externer organischer Lösungsmittel, d. h. von als Lösungsmittel wirksamen Komponenten, die von den zur Herstellung der Carbonsäureester eingesetzten Edukten und den gebildeten Reaktionsprodukten verschieden sind, kann in der Regel verzichtet werden.
- Das erfindungsgemäße Verfahren eignet sich speziell zur Herstellung von Estern der Terephthalsäure, Trimellitsäure, Benzoesäure und Estern alicyclischer und aliphatischer Carbonsäuren die aufgrund ihrer günstigen toxikologischen Eigenschaften von großer Bedeutung für den Einsatz als Weichmacher sind.
- Die erhaltenen Carbonsäureester sind nicht oder nur gering gefärbt und zeichnen sich durch eine niedrige Hazen-Farbzahl (bestimmbar nach DIN/EN/ISO 6271-2) aus. Diese ist in der Regel wenigstens gleich gut oder besser wie bei Produkten, die nach dem wesentlich aufwändigeren Verfahren mittels Katalyse durch Tetraalkyltitanate erhalten werden.

Im Rahmen der vorliegenden Erfindung wird unter dem Ausdruck "Reaktionssystem" die Gesamtheit aller der Umsetzung zugeführten Einsatzstoffe, Lösungsmittel, Katalysatoren sowie die bei der Umsetzung gebildeten Produkte und Zwischenprodukte verstanden.

Im Rahmen der vorliegenden Erfindung umfasst der Ausdruck "Alkyl" unsubstituierte geradkettige oder verzweigte Alkylgruppen, die 1 bis 30 Kohlenstoffatome, bevorzugt 1 bis 20 Kohlenstoffatome, enthalten. Besonders bevorzugt handelt es sich dabei um unsubstituierte geradkettige oder verzweigte C₁-C₁₃-Alkylgruppen, besonders bevorzugt um unsubstituierte geradkettige oder verzweigte C₁-C₆-Alkylgruppen. Zu den unsubstituierten geradkettige oder verzweigte C₁-C₁₃-Alkylgruppen zählen Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 1-Ethylpentyl, 2-Ethylpentyl, 1-Propylbutyl, 1-Ethyl-2-methylpropyl, n-Octyl, Isooctyl, 2-Ethylhexyl, n-Nonyl, Isononyl, 2-Propylhexyl, n-Decyl, Isodecyl, 2-Propylheptyl, n-Undecyl, Isoundecyl, n-Dodecyl, Isododecyl, n-Tridecyl oder Isotridecyl und dergleichen.

Der Ausdruck "Alkyl" beinhalten in seiner Definition auch die Ausdrücke "C₅-C₁₃-Alkyl" "C₁-C₁₀-Alkyl" und "C₁-C₆-Alkyl".

Die Kohlenstoffkette der Alkylreste kann durch eine oder mehrere nicht benachbarte Heteroatome oder heteroatomhaltige Gruppen unterbrochen sein. Alkylreste, deren Kohlenstoffkette durch eine oder mehrere nicht benachbarte Heteroatome oder heteroatomhaltige Gruppen unterbrochen sind, sind ausgewählt untersolchen Alkylresten, die durch -O-, -S-, -NR^{a}-, -PR^{a}-, -SiR^{a}R^{b} und/oder-SO₂- unterbrochen sind. R^{a} und R^{b} stehen vorzugsweise für Wasserstoff, Alkyl, Cycloalkyl, Heterocycloalkyl oder Aryl.

Beispiele für Alkylreste, deren Kohlenstoffketten durch eine oder zwei nicht benachbarte Heteroatome -O- unterbrochen sein können, sind die folgenden:
Methoxymethyl, Diethoxymethyl, 2-Methoxyethyl, 2-Ethoxyethyl, 2-Propoxyethyl, Diethoxyethyl, 2-Butoxyethyl, 2-Octyloxyethyl, 2-Methoxypropyl, 3-Methoxypropyl, 3-Ethoxypropyl, 3-Propoxypropyl, 2-Isopropoxyethyl, 2-Butoxypropyl, 3-Butoxypropyl, 4-Methoxybutyl, 4-Ethoxybutyl, 4-Propoxybutyl, 6-Methoxyhexyl, 3,6-Dioxa-heptyl (5-Methoxy-3-oxa-pentyl), 3,6-Dioxa-octyl (7-Methoxy-4-oxa-heptyl), 4,8-Dioxa-nonyl (7-Methoxy-4-oxa-heptyl), 3,7-Dioxa-octyl, 3,7-Dioxa-nonyl, 4,7-Dioxa-octyl, 4,7-Dioxa-nonyl, 2- und 4-Butoxybutyl, 4,8-Dioxadecyl, 9-Ethoxy-5-oxa-nonyl.

Beispiele für Alkylreste, deren Kohlenstoffketten durch drei oder mehr als drei nicht benachbarte Heteroatome -O- unterbrochen sein können, sind auch Oligo- und Polyoxyalkylene, d. h. Verbindungen mit Wiederholungseinheiten, die vorzugsweise ausgewählt sind unter (CH₂CH₂O)ₓ₁, (CH(CH₃)CH₂O)ₓ₂ und ((CH₂)₄O)ₓ₃, wobei x1, x2 und x3 unabhängig voneinander für eine ganze Zahl von 3 bis 100, vorzugsweise 3 bis 80, stehen. Die Summe aus x1, x2 und x3 steht für eine ganze Zahl von 3 bis 300, insbesondere 3 bis 100. In Polyoxyalkylenen, die zwei oder drei verschiedenartige Wiederholungseinheiten aufweisen, ist die Reihenfolge beliebig, d. h. es kann sich um statistisch verteilte, alternierende oder blockförmige Wiederholungseinheiten handeln. Beispiele hierfür sind 3,6,9-Trioxadecyl, 3,6,9-Trioxaundecyl, 3,6,9-Trioxadodecyl, 4,8,12-Trioxatridecyl (11-Methoxy-4,8-dioxa-undecyl), 4,8,12-Trioxatetradecyl, 14-Methoxy-5,10-dioxa-tetradecyl, 5,10,15-Trioxaheptadecyl, 3,6,9,12-Tetraoxatridecyl, 3,6,9,12-Tetraoxatetradecyl, 4,8,12,16-Tetraoxaheptadecyl (15-Methoxy-4,8,12-trioxapentadecyl), 4,8,12,16-Tetraoxa-octadecyl und dergleichen.

Beispiele für Alkylreste, deren Kohlenstoffketten durch eine oder mehrere, z. B. 1, 2, 3, 4 oder mehr als 4, nicht benachbarte Heteroatome -S- unterbrochen sein kann, sind die folgenden:
Butylthiomethyl, 2-Methylthioethyl, 2-Ethylthioethyl, 2-Propylthioethyl, 2-Butylthio-ethyl, 2-Dodecylthioethyl, 3-Methylthiopropyl, 3-Ethylthiopropyl, 3-Propylthiopropyl, 3-Butylthiopropyl, 4-Methylthiobutyl, 4-Ethylthiobutyl, 4-Propylthiobutyl, 3,6-Dithiaheptyl, 3,6-Dithia-octyl, 4,8-Dithia-nonyl, 3,7-Dithia-octyl, 3,7-Di-thia-nonyl, 2- und 4-Butylthiobutyl, 4,8-Dithia-decyl, 3,6,9-Trithia-decyl, 3,6,9-Trithia-undecyl, 3,6,9-Trithia-dodecyl, 3,6,9,12-Tetrathia-tridecyl und 3,6,9,12-Tetrathia-tetradecyl.

Beispiele für Alkylreste, deren Kohlenstoffketten durch eine oder zwei nicht benachbarte heteroatomhaltige Gruppen -NR^{a}- unterbrochen sind, sind die Folgenden:
2-Monomethyl- und 2-Monoethylaminoethyl, 2-Dimethylaminoethyl, 3-Methylaminopropyl, 2- und 3-Dimethylaminopropyl, 3-Monoisopropylaminopropyl, 2- und 4-Monopropylaminobutyl, 2- und 4-Dimethylaminobutyl, 6-Methylaminohexyl, 6-Dimethylaminohexyl, 6-Methyl-3,6-diazaheptyl, 3,6-Dimethyl-3,6-diazaheptyl, 3,6-Diazaoctyl und 3,6-Dimethyl-3,6-diazaoctyl.

Beispiele für Alkylreste, deren Kohlenstoffketten durch drei oder mehr als drei nicht benachbarte heteroatomhaltige Gruppen -NR^{a}- unterbrochen sein können, sind auch Oligo- und Polyalkylenimine. Das zuvor für die Polyoxyalkylene Gesagte gilt analog für Polyalkylenimine, wobei das Sauerstoffatom jeweils durch eine Gruppe NR^{a} ersetzt ist, worin R^{a} vorzugsweise für Wasserstoff oder C₁-C₄-Alkyl steht. Beispiele hierfür sind 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl, 3,6,9,12-Tetramethyl-3,6,9,12-tetraazatridecyl und dergleichen.

Beispiele für Alkylreste, deren Kohlenstoffketten durch eine oder mehrere, z. B. 1 oder 2 nicht benachbarte Gruppen -SO₂- unterbrochen sind, sind 2-Methylsulfonylethyl, 2-Ethylsulfonylethyl, 2-Propylsulfonylethyl, 2-Isopropylsulfonylethyl, 2-Buthylsulfonylethyl, 2- Methylsulfonylpropyl, 3-Methylsulfonylpropyl, 2-Ethylsulfonylpropyl, 3-Ethylsulfonylpropyl, 2- Propylsulfonylpropyl, 3-Propylsulfonylpropyl, 2-Butylsulfonylpropyl, 3-Butylsulfonylpropyl, 2-Methylsulfonylbutyl, 4-Methylsulfonylbutyl, 2- Ethylsulfonylbutyl, 4-Ethylsulfonylbutyl, 2-Propylsulfonylbutyl, 4-Propylsulfonylbutyl und 4-Butylsulfonylbutyl.

Die Alkylgruppen können in Abhängigkeit von der Länge der Alkylkette einen oder mehrere (z. B. 1, 2, 3, 4, 5 oder mehr als 5) Substituenten aufweisen. Diese sind vorzugsweise unabhängig voneinander ausgewählt unter Cycloalkyl, Cycloalkyloxy, Polycyclyl, Polycyclyloxy, Heterocycloalkyl, Aryl, Aryloxy,-Hetaryl, Halogen=O, NE¹E², Nitro und Cyano, wobei E¹ und E² unabhängig voneinander für Wasserstoff, Alkyl, Cycloalkyl, Heterocycloalkyl, Aryl oder Hetaryl stehen. Cycloalkyl-, Cycloalkyloxy, Polycycloalkyl-, Polycyclyloxy-, Heterocycloalkyl-, Aryl- und Hetarylsubstituenten der Alkylgruppen können ihrerseits unsubstituiert oder substituiert sein; geeignete Substituenten sind die nachfolgend für diese Gruppen genannten.

Die vorstehenden Ausführungen zu Alkyl gelten prinzipiell auch für die Alkylteile in Alkoxy, Alkylamino, Dialkylamino, Alkylsulfinyl, Alkylsulfonyl, etc.

Geeignete substituierte Alkylreste sind die folgenden:
Alkyl, das durch Amino substituiert ist, wie z. B. 2-Aminoethyl, 2-Aminopropyl, 3-Aminopropyl, 4-Aminobutyl, 6-Aminohexyl und dergleichen.

Alkyl, das durch Cyano substituiert ist, wie z. B. 2-Cyanoethyl, 3-Cyanopropyl, 3-Cyanobutyl und 4-Cyanobutyl;
Alkyl, das durch Halogen, wie nachfolgend definiert, substituiert ist, wobei in der Alkylgruppe die Wasserstoffatome teilweise oder vollständig durch Halogenatome ersetzt sein können, wie C₁-C₁₈-Fluoralkyl, z. B. Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl und dergleichen, C₁-C₁₈-Chloralkyl, z. B. Chlormethyl, Dichlormethyl, Trichlormethyl, 2-Chlorethyl, 2- und 3-Chlorpropyl, 2-, 3- und 4-Chlorbutyl, 1,1-Dimethyl-2-chlorethyl und dergleichen
Alkyl, das durch Nitro substituiert ist, wie z. B. 2-Nitroethyl, 2- und 3-Nitropropyl und 2-, 3- und 4-Nitrobutyl und dergleichen.

Alkyl, das durch Cycloalkyl substituiert ist, wie z. B. Cyclopentylmethyl, 2-Cyclopentylethyl, 3-Cyclopentylpropyl, Cyclohexylmethyl, 2-Cyclohexylethyl, 3-Cyclohexylpropyl und dergleichen.

Alkyl, das durch =O (Oxogruppe) substituiert ist, wie z. B. 2-Oxopropyl, 2-Oxobutyl, 3-Oxobutyl, 1-Methyl-2-oxopropyl, 2-Oxopentyl, 3-Oxopentyl, 1-Methyl-2-oxobutyl, 1-Methyl-3-oxobutyl, 2-Oxohexyl, 3-Oxohexyl, 4-Oxohexyl, 2-Oxoheptyl, 3-Oxoheptyl, 4-Oxoheptyl, 4-Oxoheptyl und dergleichen.

Der Ausdruck "Cycloalkyl" umfasst im Rahmen der vorliegenden Erfindung unsubstituierte monocyclische gesättigte Kohlenwasserstoffgruppen mit im Allgemeinen 3 bis 12 Kohlenstoffringgliedern (C₃-C₁₂-Cycloalkylgruppen) wie Cyclopropyl, Cyclobutyl, Cyclopentyl, Cyclohexyl, Cycloheptyl, Cyclooctyl, Cyclononyl, Cyclodecyl, Cycloundecyl oder Cyclododecyl, insbesondere C₅-C₁₂-Cycloalkyl. Die Cycloalkylreste können einen oder mehrere (z. B. 1, 2, 3, 4, 5 oder mehr als 5) Substituenten aufweisen. Geeignete Substituenten sind in der Regel ausgewählt unter Alkyl, den zuvor für die Alkylgruppen genannten Substituenten sowie Alkoxy. Im Falle von Halogen können die Wasserstoffatome der substituierten Cycloalkylgruppen partiell oder vollständig durch Halogen substituiert sein.

Beispiele für unsubstituierte und substituierte Cycloalkylgruppen sind Cyclopentyl, 2- und 3-Methylcyclopentyl, 2- und 3-Ethylcyclopentyl, Chlorpentyl, Dichlorpentyl, Dimethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Chlorhexyl, Dimethylcyclohexyl, Diethylcyclohexyl, Methoxycyclohexyl, Dimethoxycyclohexyl, Diethoxycyclohexyl, Butoxycyclohexyl, Methylthiocyclohexyl, Chlorcyclohexyl, Dichlorcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl, partiell fluoriertes Cycloalkyl und perfluoriertes Cycloalkyl der Formel CₙF_{2(n-a)-(1-b)}H_{2a-b} mit n = 5 bis 12, 0 <= a <= n und b = 0 oder 1.

Der Ausdruck "Cycloalkyl" beinhaltet in seiner Definition auch den Ausdruck "C₅-C₆-Cycloalkyl".

Ionische Flüssigkeiten bezeichnen im Rahmen der vorliegenden Anmeldung organische Salze, die bereits bei Temperaturen unterhalb 250 °C flüssig sind. Vorzugsweise besitzen die ionischen Flüssigkeiten einen Schmelzpunkt von weniger als 220 °C, besonders bevorzugt weniger als 200 °C, insbesondere weniger als 150 °C.
Ionische Flüssigkeiten, die bereits bei Raumtemperatur in flüssigem Aggregatszustand vorliegen, werden beispielsweise von K.N. Marsh et al., Fluid Phase Equilibria 219 (2004), 93-98 und J.G. Huddleston et al., Green Chemistry 2001, 3, 156-164 beschrieben.

In der ionischen Flüssigkeit liegen Kationen sowie Anionen vor. Dabei kann innerhalb der ionischen Flüssigkeit vom Kation ein Proton oder ein Alkylrest an das Anion übertragen werden, wodurch zwei neutrale Moleküle resultieren. In der erfindungsgemäß eingesetzten ionischen Flüssigkeit liegt in der Regel ein Gleichgewicht von Anionen, Kationen sowie daraus gebildeten neutralen Molekülen vor.

Die erfindungsgemäß eingesetzten ionischen Flüssigkeiten weisen polyatomige, d. h. mehratomige Anionen, mit zwei oder mehr als zwei Atomen auf.

Der Ausdruck "Alkoxy" steht für eine über ein Sauerstoffatom gebundene Alkylgruppe. Beispiele für Alkoxy sind: Methoxy, Ethoxy, n-Propoxy, 1-Methylethoxy, Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy, Hexoxy sowie R^{A}O-(CH₂CH₂CH₂CH₂O)ₙ-CH₂CH₂CH₂CH₂O- mit R^{A} Wasserstoff oder C₁-C₄-Alkyl, bevorzugt Wasserstoff, Methyl oder Ethyl und n 0 bis 10, bevorzugt 0 bis 3.

Der Ausdruck "Alkoxy" beinhaltet in seiner Definition auch den Ausdruck "C₁-C₆-Alkoxy".

Alkylsulfinyl steht für eine über eine S(=O)-Gruppe gebundene Alkylgruppe.

Alkylsulfonyl steht für eine über eine S(=O)₂-Gruppe gebundene Alkylgruppe.

Durch Aryl substituierte Alkylreste ("Arylalkyl") weisen wenigstens eine, wie nachfolgend definierte, unsubstituierte oder substituierte Arylgruppe auf. Geeignete Substituenten an der Arylgruppe sind die nachfolgend genannten. Dabei kann die Alkylgruppe in "Arylalkyl" wenigstens einen weiteren Substituenten, wie vorstehend definiert, tragen und/oder durch eine oder mehrere nicht benachbarte Heteroatome oder heteroatomhaltige Gruppen, die ausgewählt sind unter -O-, -S-, -NR^{a}-, und/oder - SO₂- unterbrochen sein. Arylalkyl steht vorzugsweise für Phenyl-Ci-Cio-alkyl, besonders bevorzugt für Phenyl-C₁-C₄-alkyl, z. B. für Benzyl, 1-Phenethyl, 2-Phenethyl, 1-Phenprop-1-yl, 2-Phenprop-1-yl, 3-Phenprop-1-yl, 1-Phenbut-1-yl, 2-Phenbut-1-yl, 3-Phenbut-1-yl, 4-Phenbut-1-yl, 1-Phenbut-2-yl, 2-Phenbut-2-yl, 3-Phenbut-2-yl, 4-Phenbut-2-yl, 1-(Phenmeth)-eth-1-yl, 1-(Phenmethyl)-1-(methyl)-eth-1-yl oder-(Phenmethyl)-1-(methyl)-prop-1-yl; vorzugsweise für Benzyl und 2-Phenethyl.

Der Ausdruck "Alkenyl" umfasst im Sinne der vorliegenden Erfindung unsubstituierte geradkettige und verzweigte Alkenylgruppen, die in Abhängigkeit von der Kettenlänge eine oder mehrere Doppelbindungen (z. B. 1, 2, 3, 4 oder mehr als 4) tragen können. Bevorzugt sind C₂-C₁₈-, besonders bevorzugt C₂-C₁₀-Alkenylgruppen. Alkenyl steht beispielsweise für Ethenyl (Vinyl), 1-Propenyl, 2-Propenyl, 1-Methylethenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl und dergleichen.

Die Alkenylgruppen können einen oder mehrere (z. B. 1, 2, 3, 4, 5 oder mehr als 5) Substituenten aufweisen. Geeignete Substituenten sind z. B. ausgewählt unter =O, Cycloalkyl, Cycloalkyloxy, Polycyclyl, Heterocycloalkyl, Aryl, Hetaryl, Halogen, Alkylsulfinyl, Alkylsulfonyl, NE³E⁴, Nitro und Cyano, wobei E³ und E⁴ unabhängig voneinander für Wasserstoff, Alkyl, Cycloalkyl, Heterocycloalkyl, Aryl oder Hetaryl stehen.

Die Kohlenstoffkette der Alkenylreste kann durch eine oder mehrere nicht benachbarte Heteroatome oder heteroatomhaltige Gruppen, die vorzugsweise ausgewählt sind unter-O-, -S-, -NR^{a}- und/oder -SO₂-, unterbrochen sein.

Der Ausdruck "Alkenyl" beinhalten in seiner Definition auch die Ausdrücke "C₁-C₁₀-Alkenyl" und "C₁-C₆-Alkenyl".

Cycloalkyloxy steht für eine über Sauerstoff gebundene Cycloalkylgruppe, wie vorstehend definiert.

Der Ausdruck "Cycloalkenyl" umfasst unsubstituierte, einfach oder zweifach ungesättigte Kohlenwasserstoffgruppen mit 3 bis 5, bis 8 bis 12, vorzugsweise 5 bis 12 Kohlenstoffringgliedern, wie Cyclopent-1-en-1-yl, Cyclopent-2-en-1-yl, Cyclopent-3-en-1-yl, Cyclohex-1-en-1-yl, Cyclohex-2-en-1-yl, Cyclohex-3-en-1-yl, Cyclohexa-2,5-dien-1-yl und dergleichen. Geeignete Substituenten sind die zuvor für Cycloalkyl genannten.

Cycloalkenyloxy steht für eine über Sauerstoff gebundene Cycloalkenylgruppe, wie vorstehend definiert.

Der Ausdruck "Polycyclyl" umfasst im Rahmen der vorliegenden Erfindung im weitesten Sinn Verbindungen, die wenigstens zwei Ringe enthalten, unabhängig davon, wie diese Ringe verknüpft sind. Hierbei kann es sich um carbocyclische und/oder heterocyclische Ringe handeln. Die Ringe können gesättigt oder ungesättigt sein. Die Ringe können über Einfach- oder Doppelbindung verknüpft ("mehrkernige Verbindungen"), durch Annelierung verbunden ("kondensierte Ringsysteme") oder überbrückt ("überbrückte Ringsysteme", "Käfigverbindungen") sein. Bevorzugte polycyclische Verbindungen sind überbrückte Ringsysteme und kondensierte Ringsysteme. Kondensierte Ringsysteme können durch Annelierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme. Zu den überbrückten Ringsystemen zählen im Rahmen der vorliegenden Erfindung solche, die nicht zu den mehrkernigen Ringsystemen und nicht zu den kondensierten Ringsystemen zählen und bei denen mindestens zwei Ringatome zumindest zwei verschiedenen Ringen angehören. Bei den überbrückten Ringsystemen unterscheidet man je nach Anzahl der Ringöffnungsreaktionen, die formal erforderlich sind, um zu einer offenkettigen Verbindung zu gelangen, Bi-, Tri-, Tetracyclo- Verbindungen usw., die aus zwei, drei, vier usw. Ringen bestehen. Der Ausdruck "Bicycloalkyl" umfasst dabei bicyclische Kohlenwasserstoffreste mit vorzugsweise 5 bis 10 C-Atomen wie Bicyclo[2.2.1]hept-1-yl, Bicyclo[2.2.1]hept-2-yl, Bicyclo[2.2.1]hept-7-yl, Bicyclo[2.2.2]oct-1-yl, Bicyclo[2.2.2]oct-2-yl, Bicyclo[3.3.0]octyl, Bicyclo[4.4.0]decyl und dergleichen. Der Ausdruck "Bicycloalkenyl" umfasst einfach ungesättigte, bicyclische Kohlenwasserstoffreste mit vorzugsweise 5 bis 10 C-Atomen, wie Bicyclo[2.2.1]hept-2-en-1-yl.

Der Ausdruck "Aryl" umfasst im Rahmen der vorliegenden Erfindung ein- oder mehrkernige aromatische Kohlenwasserstoffreste. Aryl steht in der Regel für Kohlenwasserstoffreste mit 6 bis 10, bis 14, bis 18, vorzugsweise 6 bis 10 Kohlenstoffringgliedern. Aryl steht vorzugsweise für unsubstituiertes oder substituiertes Phenyl, Naphthyl, Anthracenyl, Phenanthrenyl, Naphthacenyl, Chrysenyl, Pyrenyl, etc., und besonders bevorzugt für Phenyl oder Naphthyl. Die Arylgruppen können in Abhängigkeit von der Anzahl und Größe ihrer Ringsysteme einen oder mehrere (z. B. 1, 2, 3, 4, 5 oder mehr als 5) Substituenten aufweisen. Diese sind vorzugsweise unabhängig voneinander ausgewählt unter Alkyl, Alkoxy, Cycloalkyl, Cycloalkyloxy, Heterocycloalkyl, Aryl, Aryloxy, Hetaryl, Halogen, Alkylsulfinyl, Alkylsulfonyl, NE⁵E⁶, Nitro und Cyano, wobei E⁵ und E⁶ unabhängig voneinander für Wasserstoff, Alkyl, Cycloalkyl, Cycloalkyloxy, Polycyclylyl, Polycyclyloxy, Heterocycloalkyl, Aryl, Aryloxy oder Hetaryl stehen. Besonders bevorzugt steht Aryl für Phenyl, das im Falle einer Substitution im Allgemeinen 1, 2, 3, 4 oder 5, vorzugsweise 1, 2 oder 3 Substituenten tragen kann.

Aryl, das einen oder mehrere Reste trägt, steht beispielsweise für 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl, 2,4,6-Tri-tert.-butylphenyl und 2-, 3-, 4-Dodecylphenyl; 2-, 3- und 4-Methoxyphenyl, 2,4-, 2,5-, 3,5- und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl, 2-, 3- und 4-Butoxyphenyl, 2-, 3-, 4-Hexyloxyphenyl; 2-, 3-, 4-Chlorphenyl, 2,4-, 2,5-, 3,5- und 2,6-Dichlorphenyl, Trichlorphenyl, 2-, 3-, 4-Fluorphenyl, 2,4-, 2,5-, 3,5- und 2,6-Difluorphenyl, Trifluorphenyl, wie z. B. 2,4,6-Trifluorphenyl, Tetrafluorphenyl, Pentafluorphenyl, 2-, 3- und 4-Cyanophenyl; 2-Nitrophenyl, 4-Nitrophenyl, 2,4-Dinitrophenyl, 2,6-Dinitrophenyl; 4-Dimethylaminophenyl; 4-Acetylphenyl; Methoxyethylphenyl, Ethoxymethylphenyl; Methylthiophenyl, Isopropylthiophenyl oder tert.-Butylthiophenyl; Methylnaphthyl; Isopropylnaphthyl oder Ethoxynaphthyl. Beispiele für substituiertes Aryl, worin zwei Substituenten, die an benachbarte Kohlenstoffatome des Arylrings gebunden sind, einen kondensierten Ring oder kondensiertes Ringsystem bilden, sind Indenyl und Fluorenyl.

Der Ausdruck "Aryloxy" steht im Rahmen der vorliegenden Erfindung für über ein Sauerstoffatom gebundenes Aryl.

Der Ausdruck "Heterocycloalkyl" umfasst im Rahmen der vorliegenden Erfindung unsubstituierte, nicht-aromatische, ungesättigte oder vollständig gesättigte, cycloaliphatische Gruppen mit im Allgemeinen 5 bis 8 Ringatomen, vorzugsweise 5- oder 6 Ringatomen, in denen 1, 2 oder 3 der Ringkohlenstoffatome durch Heteroatome, ausgewählt unter Sauerstoff, Stickstoff, Schwefel und einer Gruppe -NR^{a}- ersetzt sind. Heterocycloalkylgruppen können mit einer oder mehreren, beispielsweise 1, 2, 3, 4, 5 oder 6, C₁-C₆-Alkylgruppen substituiert sein. Beispielhaft für heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothienyl, Dihydrothienyl, Tetrahydrofuranyl, Dihydrofuranyl, Tetrahydropyranyl, 1,2-Oxazolin-5-yl, 1,3-Oxazolin-2-yl und Dioxanyl genannt. Stickstoffhaltiges Heterocycloalkyl kann prinzipiell sowohl über ein Kohlenstoffatom als auch über ein Stickstoffatom gebunden sein.

Der Ausdruck "Heteroaryl (Hetaryl)" umfasst im Rahmen der vorliegenden Erfindung unsubstituierte, heteroaromatische, ein- oder mehrkernige Gruppen, mit im Allgemeine 5 bis 14 Ringatomen, vorzugsweise 5 oder 6 Ringatomen, in denen 1, 2 oder 3 der Ringkohlenstoffatome durch ein, zwei, drei oder vier Heteroatome, ausgewählt unter O, N, -NR^{a}- und S ersetzt sind. Beispiele für Heteroarylgruppen sindFuryl, Thienyl, Oxazolyl, Isoxazolyl, Thiazolyl, Isothiazolyl, Benzofuranyl, Benzthiazolyl, Benzimidazolyl, Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl. Die heterocycloaromatischen Gruppen können im Allgemeinen 1, 2 oder 3 Substituenten tragen. Die Substituenten sind in der Regel ausgewählt unter C₁-C₆-Alkyl, C₁-C₆-Alkoxy, Hydroxy, Carboxy, Halogen und Cyano.

5- bis 7-gliedrige Stickstoff enthaltende Heterocycloalkyl- oder Heteroarylreste, die gegebenenfalls weitere Heteroatome enthalten, stehen beispielsweise für Pyrrolyl, Pyrazolyl, Imidazolyl, Triazolyl, Pyrrolidinyl, Pyrazolinyl, Pyrazolidinyl, Imidazolinyl, Imidazolidinyl, Pyridinyl, Pyridazinyl, Pyrimidinyl, Pyrazinyl, Triazinyl, Piperidinyl, Piperazinyl, Oxazolyl, Isooxazolyl, Thiazolyl, Isothiazolyl, Indolyl, Chinolinyl, Isochinolinyl oder Chinaldinyl.

Halogen steht für Fluor, Chlor, Brom oder lod.

Der Ausdruck "Acyl" steht im Sinne der vorliegenden Erfindung für Alkanoyl-, Hetaroyl- oder Aroylgruppen mit im Allgemeinen 1 bis 11, vorzugsweise 2 bis 8 Kohlenstoffatomen, beispielsweise für die Formyl-, Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Reste E¹ und E², E³ und E⁴, E⁵ und E⁶ sind unabhängig voneinander ausgewählt unter Wasserstoff, Alkyl, Cycloalkyl und Aryl. Die Gruppen NE¹E², NE³E⁴ und NE⁵E⁶ stehen vorzugsweise für N,N-Dimethylamino, N,N-Diethylamino, N,N-Dipropylamino, N,N-Diisopropylamino, N,N-Di-n-butylamino, N,N-Di-tert.-butylamino, N,N-Dicyclohexylamino oder N,N-Diphenylamino.

Bei dem Anion [Y]^{m-} geeigneter ionischer Flüssigkeiten handelt es sich um ein m-wertiges mehratomiges Anion, wobei m die Werte 1, 2, 3 oder 4 aufweist.

Geeignete Anionen [Y]^{m-} sind ausgewählt unter:
- der Gruppe der Pseudohalogenide und halogenhaltigen Verbindungen der Formeln:
   BF₄⁻, PF₆⁻, CF₃SO₃⁻, (CF₃SO₃)₂N⁻, CN-, SCN-, OCN-; c
- der Gruppe der Sulfonate der allgemeinen Formel:
   R^{c}SO₃⁻;
- der Gruppe der Phosphite der allgemeinen Formeln:
   PO₃³⁻, R^{c}PO₃²⁻, R^{c}R^{d}PO₃⁻;
- der Gruppe der Phosphonite und Phosphinite der allgemeinen Formeln:
   R^{c}R^{d}PO₂⁻, R^{c}R^{d}PO⁻;
- der Gruppe der Borate der allgemeinen Formeln:
   BO₃³⁻, HBO₃²⁻, H₂BO₃⁻, R^{c}R^{d}BO₃⁻, R^{c}HBO₃⁻, R^{c}BO₃²⁻, B(OR^{c})(OR^{d})(OR^{e})(OR^{f})⁻, B(HSO₄)₄⁻, B(R^{c}SO₄)₄⁻;
- der Gruppe der Boronate der allgemeinen Formeln:
   R^{c}BO₂²⁻, R^{c}R^{d}BO⁻
- der Gruppe der Silikate und Kieselsäuresäureester der allgemeinen Formeln: SiO₄⁴⁻, HSiO₄³⁻, H₂SiO₄²⁻, H₃SiO₄⁻, R^{c}SiO₄³⁻, R^{c}R^{d}SiO₄²⁻, R^{c}R^{d}R^{e}SiO₄⁻, HR^{c}SiO₄²⁻, H₂R^{c}SiO₄⁻, HR^{c}R^{d}SiO₄⁻;
- der Gruppe der Alkyl- bzw. Arylsilanolate der allgemeinen Formeln:
   R^{c}SiO₃³⁻, R^{c}R^{d}SiO₂²⁻, R^{c}R^{d}R^{e}SiO⁻, R^{c}R^{d}R^{e}SiO₃⁻, R^{c}R^{d}R^{e}SiO₂⁻, R^{c}R^{d}SiO₃²⁻;
- der Gruppe der Carbonsäureimide, Bis(sulfonyl)imide und Sulfonylimide der allgemeinen Formeln: 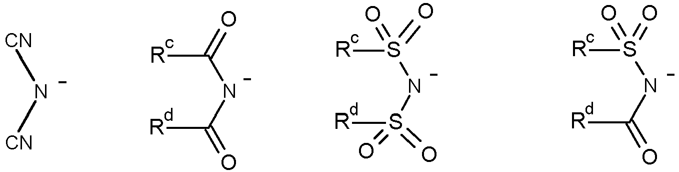
- der Gruppe der Methide der allgemeinen Formel: 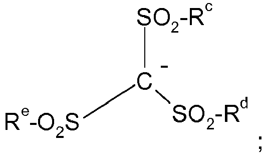
- der Gruppe der Hydrogensulfide, Polysulfide und Hydrogenpolysulfide der allgemeinen Formeln:
   [Sᵥ]²⁻, [HSᵥ]⁻, wobei v eine ganze positive Zahl von 2 bis 10 ist,
wobei die Rest R^{c}, R^{d}, R^{e} und R^{f} unabhängig voneinander ausgewählt sind unter Wasserstoff, unsubstituiertes oder substituiertes Alkyl, Cycloalkyl, Heterocycloalkyl, Aryl, Heteroaryl,
wobei in Anionen, die mehrere Reste R^{c} bis R^{f} aufweisen, auch jeweils zwei dieser Reste zusammen mit dem Teil des Anions, an das sie gebunden sind, für wenigstens einen, gesättigten, ungesättigten oder aromatischen Ring oder ein Ringsystem mit 1 bis 12 Kohlenstoffatomen stehen können, wobei der Ring oder das Ringsystem 1 bis 5 nicht benachbarte Heteroatome oder heteroatomhaltige Gruppen aufweisen kann, die vorzugsweise ausgewählt sind unter Sauerstoff, Stickstoff, Schwefel und NR^{a}, und wobei der Ring oder das Ringsystem unsubstituiert ist oder substituierten sein kann.

Besonders geeignete Anionen sind ausgewählt unter der Gruppe der Pseudohalogenide und halogenhaltigen Verbindungen, der Gruppe der Sulfonate sowie der Gruppe der Phosphate.

Ganz besonders geeignete Anionen sind BF₄⁻, PF₆⁻, CF₃SO₃⁻, SCN-, PO₃³⁻, R^{c}PO₃²⁻ und R^{c}SO₃⁻, wobei R^{c} die oben genannte Definition aufweist.

Besonders geeignete ionische Flüssigkeiten sind ausgewählt unter Salzen der allgemeinen Formel (la), 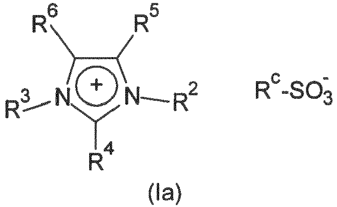 worin
Reste R² und R³ unabhängig voneinander für Wasserstoff, unsubstituiertes oder substituiertes Alkyl, Alkenyl, Cycloalkyl, Cycloalkenyl, Aryl oder Heteroaryl stehen,
Reste R⁴, R⁵, und R⁶ unabhängig voneinander für Wasserstoff, unsubstituiertes oder substituiertes Alkyl, Alkenyl, Cycloalkyl, Cycloalkenyl, Heterocycloalkyl, Aryl, Heteroaryl oder Alkoxy stehen,
   und
R^{c} für Alkyl, Cycloalkyl oder Aryl steht, wobei Aryl mit 1, 2, oder 3 Alkylresten substituiert sein kann.

In dem erfindungsgemäßen Verfahren ist die wenigstens eine ionische Flüssigkeit ausgewählt unter Salzen der allgemeinen Formel (la), worin
Reste R² und R³ unabhängig voneinander für Wasserstoff, unsubstituiertes C₁-C₆-Alkyl, unsubstituiertes C₂-C₆-Alkenyl, unsubstituiertes C₅-C₆-Cycloalkyl, C₆-C₁₀-Aryl oder Heteroaryl mit 5 bis 6 Ringatomen, stehen, wobei die 2 letztgenannten Reste auch 1, 2 oder 3 Substituenten, ausgewählt unter C₁-C₆-Alkyl, C₁-C₆-Alkoxy oder Halogen, aufweisen können,
Reste R⁴, R⁵, und R⁶ unabhängig voneinander für Wasserstoff, unsubstituiertes C₁-C₆-Alkyl, unsubstituiertes C₅-C₆-Cycloalkyl oder Benzyl stehen, wobei Benzyl auch 1, 2 oder 3 Substituenten, ausgewählt unter C₁-C₆-Alkyl, C₁-C₆-Alkoxy oder Halogen, aufweisen kann,
und
R^{c} für unsubstituiertes C₁-C₆-Alkyl, unsubstituiertes C₅-C₆-Cycloalkyl oder Aryl steht, wobei Aryl mit 1, 2, oder 3 C₁-C₆-Alkylresten substituiert sein kann.

Speziell steht in den Verbindungen der allgemeinen Formel (la) die Reste R² und R³ unabhängig voneinander für Wasserstoff oder unsubstituiertes C₁-C₆-Alkyl und die Reste R⁴, R⁵, und R⁶ für Wasserstoff.

Geeignete ionische Flüssigkeiten für den Einsatz in dem erfindungsgemäßen Verfahren sind kommerziell erhältlich, z.B. unter dem Markennamen Basionic® der Firma BASF SE. Vorteilhaft für einen Einsatz in dem erfindungsgemäßen Verfahren sind z.B.: 1-Ethyl-3-methylimidazoliummethansulfonat (EMIM CH₃SO₃, Basionic ST 35), 1-Butyl-3-methylimidazoliummethansulfonat (BMIM CH₃SO₃, Basionic ST 78), 1-Ethyl-3-methylimidazolium thiocyanat (BASIONIC™ VS 01) oder 1-Butyl-3-methylimidazolium thiocyanat (BASIONIC™ VS 02).

Besonders geeignete ionische Flüssigkeiten für den Einsatz in dem erfindungsgemäßen Verfahren sind z.B. 1-Ethyl-3-methylimidazoliummethansulfonat (EMIM CH₃SO₃, Basionic ST 35) und 1-Butyl-3-methylimidazoliummethansulfonat (BMIM CH₃SO₃, Basionic ST 78).

In einer speziellen Ausführungsform entspricht das Anion der in dem erfindungsgemäßen Verfahren eingesetzten wenigstens einen ionischen Flüssigkeit der deprotonierten Form der als Katalysator eingesetzten organischen Sulfonsäure. Wird beispielsweise Methansulfonsäure als Katalysator eingesetzt, ist das Anion der eingesetzten ionischen Flüssigkeit Methylsulfonat.

In der Regel wird die Umsetzung der wenigstens einen Carbonsäure und/oder des wenigstens einen Carbonsäureanhydrids und des wenigstens einen Alkohols R¹-OH, in wenigstens einem Reaktor durchgeführt.

Falls die Umsetzung in mehreren Reaktoren durchgeführt wird, sind die Reaktoren vorzugsweise hintereinander geschaltet. Das erfindungsgemäße Verfahren kann diskontinuierlich oder kontinuierlich durchgeführt werden, wird aber vorzugsweise kontinuierlich durchgeführt.

Bei den Reaktoren kann es sich um beliebige Reaktoren handeln, die zur Durchführung von chemischen Umsetzungen in flüssiger Phase geeignet sind.

Als Reaktoren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Es können aber auch Kombinationen aus aufeinander folgenden rückvermischten Reaktoren und nicht rückvermischten Reaktoren verwendet werden.

Gegebenenfalls können auch mehrere Reaktoren in einer mehrstufigen Apparatur zusammengefasst werden. Solche Reaktoren sind zum Beispiel Schlaufenreaktoren mit eingebauten Siebböden, kaskadierte Behälter, Rohrreaktoren mit Zwischeneinspeisung oder Rührkolonnen.

Vorzugsweise werden Rührkesselreaktoren verwendet. Die Rührkesselreaktoren bestehen meist aus metallischen Werkstoffen, wobei Edelstahl bevorzugt ist. Der Reaktionsansatz wird vorzugsweise mit Hilfe eines Rührers oder einer Umlaufpumpe intensiv vermischt.

In einer bevorzugten Ausführungsform wird das erfindungsgemäße Verfahren in einem einzelnen Rührkessel durchgeführt. In einer weiteren bevorzugten Ausführungsform wird das erfindungsgemäße Verfahren in wenigstens zwei Rührkesseln durchgeführt, die miteinander in Form einer Kaskade verbunden sind. Speziell bei kontinuierlicher Verfahrensführung kann es für eine möglichst vollständige Umsetzung zweckmäßig sein, mehrere Reaktoren in Form einer Kaskade zu verbinden. Die einzelnen Reaktoren werden vom Reaktionsgemisch nacheinander durchlaufen, wobei der Ablauf des ersten Reaktors dem zweiten Reaktor, der Ablauf des zweiten Reaktors dem dritten Reaktor usw. zugeführt wird. Die Kaskade kann z. B. 2 bis 10 Reaktoren umfassen, wobei 2, 3, 4 oder 5 Reaktoren bevorzugt sind.

Bei diskontinuierlicher Verfahrensdurchführung können Carbonsäure und/oder Carbonsäureanhydrid und Alkohol R¹-OH sowie die ionische Flüssigkeit und der Katalysator gleichzeitig oder nacheinander in den Reaktor eingefüllt werden. Der Katalysator kann in reiner Form oder als Lösung, bevorzugt gelöst in einem der Einsatzstoffe und/oder der ionischen Flüssigkeit, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Ester-carbonsäuren (Halbestern), beispielsweise Phthalsäureanhydrid zum Phthalsäure-monoester. Daher ist ein Katalysator häufig erst nach dem ersten Reaktionsschritt erforderlich. Aufgrund der lösevermittelnden Eigenschaften ist es in der Regel vorteilhaft, die ionische Flüssigkeit zu Beginn der Reaktion zuzugeben.

Bei kontinuierlicher Verfahrensdurchführung führt man Ströme der Edukte, der ionischen Flüssigkeit und des Katalysators in den Reaktor bzw. bei Verwendung einer Reaktorkaskade vorzugsweise in den ersten Reaktor der Kaskade ein. Die Verweilzeit im Reaktor bzw. den einzelnen Reaktoren wird dabei durch das Volumen der Reaktoren und den Mengenstrom der Edukte bestimmt.

In einer bevorzugten Ausführungsform erfolgt das erfindungsgemäße Verfahren unter Zuführung eines unter den Reaktionsbedingungen inerten Gases zum Reaktionssystem. Dazu kann das inerte Gas in den Gasraum des Reaktionssystems oder in das flüssige Reaktionsgemisch geleitet werden. Bevorzugt erfolgt die Zuführung des inerten Gases zum Reaktionssystem so, dass eine große Austauschfläche zwischen dem flüssigen Reaktionsgemisch und dem inerten Gas geschaffen wird. Die Behandlung mit dem inerten Gas während der Umsetzung hat einen Stripp-Effekt und vervollständigt die Entfernung des Reaktionswassers. Weiterhin ist es möglich, durch Zuführung eines erhitzten inerten Gases Energie in das Reaktionssystem einzutragen. In dieser Ausführung kann der Energieeintrag über den Reaktormantel entsprechend gedrosselt werden. Dadurch kann vorteilhafterweise eine Überhitzung des Reaktionsgemisches in der Nähe des Reaktormantels und die Bildung von Nebenprodukten verringert werden.

In einer besonders bevorzugten Ausführungsform wird das inerte Gas unter der Flüssigkeitsoberfläche in das siedende Reaktionsgemisch eingeführt, so dass es das Reaktionsgemisch durchperlt. Der Druck des inerten Gases muss ausreichend hoch sein, um den hydrostatischen Druck des Reaktionsgemisches oberhalb der Inertgas-Einspeisung zu überwinden. Z. B. kann man das inerte Gas 20 bis 50 cm unterhalb der Flüssigkeitsoberfläche des Reaktionsgemisches einführen.

Das inerte Gas kann über beliebige geeignete Vorrichtungen eingespeist werden. Dazu zählen z. B. Begasungslanzen oder Düsen. Die Düsen können am oder in der Nähe des Reaktorbodens vorgesehen sein. Die Düsen können hierzu als Öffnungen einer den Reaktor umgebenden Hohlkammer ausgebildet sein. Alternativ können Tauchdüsen mit geeigneten Zuleitungen eingesetzt werden. Mehrere Düsen können z. B. in Form eines Kranzes angeordnet sein. Die Düsen können nach oben oder nach unten weisen. Die Düsen weisen vorzugsweise schräg nach unten.

Vorzugsweise wird das Reaktionsgemisch durchmischt, um einen Austausch von Reaktionsgemisch im Reaktorbereich unterhalb der Einspeisung des inerten Gases mit Reaktionsgemisch im Reaktorbereich oberhalb der Einspeisung des inerten Gases zu bewirken. Zur Vermischung eignen sich beispielsweise Rührer oder Umlaufpumpe. In einer speziellen Variante wird ein sogenannter Begasungsrührer zur Zuführung des inerten Gases und zur Vermischung des Reaktionsgemischs eingesetzt.

Wird das erfindungsgemäße Verfahren in wenigstens zwei Rührkesseln durchgeführt, die miteinander in Form einer Kaskade verbunden sind, so werden vorzugsweise alle Reaktoren der Kaskade von dem inerten Gas durchströmt. Wenn mehr als ein Reaktor mit dem inerten Gas behandelt wird, kann dieses parallel zu den einzelnen Reaktoren geführt werden oder das inerte Gas passiert mehrere Reaktoren nacheinander. Es sind auch Kombinationen denkbar, in denen zwei oder mehrere Reaktoren mit frischem inerten Gas durchperlt werden und der das inerte Gas enthaltende Dampf aus wenigstens einem der Reaktoren durch wenigstens einen weiteren Reaktor geleitet wird.

Beispielsweise kann man in einer Kaskade von n Reaktoren frisches inertes Gas in den in Stromrichtung letzten Reaktor einführen, den das inerte Gas enthaltenden Brüden aus dem n-ten Reaktor sammeln und dampfförmig in das Reaktionsgemisch im (n-ersten) Reaktor einführen, usw.

Die Veresterung erfolgt erfindungsgemäß in Gegenwart eines inerten Gases. Unter einem inerten Gas wird ein Gas verstanden, welches unter den gegebenen Verfahrensbedingungen keine Reaktionen mit den an der Reaktion beteiligten Edukten, Reagenzien, Lösungsmitteln oder den entstehenden Produkten eingeht. Geeignete inerte Gase sind z. B. Stickstoff, Helium, Argon, etc. Bevorzugt wird als inertes Gas Stickstoff eingesetzt.

Erfindungsgemäß erfolgt das Verfahren unter destillativer Abtrennung wenigstens eines Teils des während der Reaktion gebildeten Wassers in Form einer azeotropen Mischung mit dem eingesetzten Alkohol R¹-OH, der anschließend zumindest teilweise in das Reaktionssystem zurückgeführt wird. Dazu entnimmt man aus dem Reaktionssystem einen Brüden, kondensiert diesen, trennt das Kondensat in eine wässrige Phase und eine Alkoholphase und führt die Alkoholphase zumindest teilweise in das Reaktionssystem zurück. "Rückführung in das Reaktionssystem" bedeutet, dass die Alkoholphase in wenigstens einen beliebigen Reaktor des Reaktionssystems geleitet wird.

Zur Kondensation bzw. partiellen Kondensation des Brüden können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Das erhaltene Kondensat wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder als Strippwasser bei der Nachbehandlung des Esters verwendet werden.

Der Brüden aus den einzelnen Reaktoren einer Kaskade kann vereinigt werden und gemeinsam kondensiert werden. Gegebenenfalls kann man jeweils mehrere Reaktoren der Kaskade zu einer Untereinheit zusammenfassen, wobei dann jeweils die Untereinheiten mit einem Kondensator gekoppelt sind. Es besteht daneben weiterhin die Möglichkeit, jeden Reaktor der Kaskade mit einem Kondensator zu koppeln.

Die zurückzuführende Alkoholphase kann in einen beliebigen Reaktor einer Kaskade geleitet oder auf mehrere Reaktoren der Kaskade aufgeteilt werden. Es ist jedoch bevorzugt, die zurückzuführende Alkoholphase nicht in den letzten Reaktor der Kaskade zu leiten. Vorzugsweise leitet man die zurückzuführende Alkoholphase ausschließlich oder überwiegend in den ersten Reaktor der Kaskade.

Für die Rückführung der Alkoholphase in das Reaktionssystem gibt es verschiedene Möglichkeiten. Eine Möglichkeit ist, die organische Phase, gegebenenfalls nach Erwärmen, in das flüssige Reaktionsgemisch zu pumpen.

Zur thermischen Optimierung des Verfahrens kann man die Alkoholphase über eine Kolonne (so genannte Rückalkohol-Kolonne) in das Reaktionssystem zurückführen. In dieser Rückalkohol-Kolonne wird die rückgeführten Alkoholphase zumindest einem Teil des Brüden entgegengeführt. Zweckmäßigerweise führt man die Alkoholphase am Kopf oder im oberen Bereich in die Rückalkohol-Kolonne ein. Das ablaufende Kondensat der Rückalkohol-Kolonne gelangt in das Reaktionssystem zurück. Bei Verwendung einer Reaktorkaskade wird das ablaufende Kondensat der Rückalkohol-Kolonne vorzugsweise in den ersten Reaktor eingeführt. Die Rückführung der Alkoholphase über die Rückalkohol-Kolonne weist den Vorteil auf, dass die rückgeführte Alkoholphase vorerwärmt und von Wasserspuren befreit wird, die nach der Phasentrennung in der organischen Phase verblieben sind bzw. gemäß ihrer thermodynamischen Löslichkeit in der organischen Phase gelöst sind. Bei der Rückalkohol-Kolonne kann es sich beispielsweise um eine Bodenkolonne, Packungskolonne oder Füllkörperkolonne handeln. Eine geringe Trennstufenzahl ist im Allgemeinen ausreichend. Geeignet ist z. B. eine Kolonne mit 2 bis 10 theoretischen Trennstufen. Bei Verwendung einer Reaktorkaskade verlässt der Brüden vorzugsweise zumindest den ersten Reaktor über die Rückalkohol-Kolonne. Ein oder mehrere oder alle weiteren Reaktoren können ebenfalls einen Brüdenabzug zur Rückalkohol-Kolonne aufweisen.

Der Alkohol R¹-OH wird vorzugsweise im stöchiometrischen Überschuss gegenüber den Carboxylgruppen eingesetzt. Dabei wird angenommen, dass ein Carbonsäureanhydrid zwei zu veresternde Carboxylgruppen aufweist. Der Alkohol R¹-OH wird besonders bevorzugt in einem 1 bis 100%igen molaren Überschuss, insbesondere in einem 5 bis 50%igen molaren Überschuss, speziell in einem 7 bis 15%igen molaren Überschuss eingesetzt.

Der Katalystor wird vorzugsweise in einer Menge von 0,5 bis 5 Mol%, besonders bevorzugt von 1 bis 2 Mol%, bezogen auf die Molmenge der Carboxylgruppen eingesetzt.

Bevorzugt ist der Veresterungskatalysator ausgewählt unter Methansulfonsäure und Toluolsulfonsäure. Insbesondere wird als Veresterungskatalysator Methansulfonsäure eingesetzt. Der Katalysator kann als Reinsubstanz oder in Form einer wässrigen Lösung eingesetzt werden.

Im Rahmen der vorliegenden Erfindung bezeichnet der Ausdruck "Gesamtchlorgehalt" die Summe aus dem Gehalt an freiem sowie organisch oder anorganisch gebundenem Chlor.

Bevorzugt weist die eingesetzte Methansulfonsäure einen Gesamtchlorgehalt von höchstens 20 ppm, bevorzugt von höchstens 5 ppm, insbesondere von höchstens 1 ppm, auf.

Bevorzugt weist die eingesetzte Methansulfonsäure einen Sulfatgehalt von höchstens 50 ppm, bevorzugt von höchstens 20 ppm, auf.

Eine besonders geeignete reine Methansulfonsäure ist erhältlich nach dem Verfahren wie in der WO 0050351 beschrieben. Eine solche reine MSA ist kommerziell unter der Bezeichnung Lutropur® der BASF SE erhältlich, entweder als 70%ige wässrige Lösung (Lutropur® MSA), oder als wasserfreie MSA (Lutropur® MSA100).

Die Veresterung wird bevorzugt in einem Temperaturbereich von 60 bis 250 °C, insbesondere von 120 bis 240 °C, durchgeführt.

Die optimalen Temperaturen hängen von den Einsatzstoffen, dem Reaktionsfortschritt und der Katalysatorkonzentration ab. Sie können für jeden Einzelfall durch Versuche leicht ermittelt werden. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktor eingestellt werden. Bei niedrig siedenden Alkoholen kann daher die Umsetzung bei Überdruck oder Umgebungsdruck und bei höher siedenden Alkoholen bei vermindertem Druck durchgeführt werden.

Wird zur Veresterung eine Kaskade aus mehreren Reaktoren eingesetzt, so können alle Reaktoren einer Kaskade bei gleicher Temperatur betrieben werden. Im Allgemeinen ist es aber bevorzugt, die Temperatur vom ersten zum letzten Reaktor einer Kaskade stetig zu erhöhen, wobei ein Reaktor bei gleicher oder höherer Temperatur betrieben wird, als der in Fließrichtung des Reaktionsgemisches stromaufwärts gelegene Reaktor. Zweckmäßigerweise können alle Reaktoren bei im Wesentlichen gleichem Druck betrieben werden.

Die Veresterung erfolgt vorzugsweise bei Umgebungsdruck oder unter vermindertem Druck. Bevorzugt wird die Veresterung bei einem Druck von 0,001 bis 2,0 bar, besonders bevorzugt 0,01 bis 1,1 bar durchgeführt.

Die Veresterung kann in Abwesenheit eines externen Lösungsmittels oder in Gegenwart eines organischen Lösungsmittels durchgeführt werden. Bevorzugt wird die Veresterung in Abwesenheit eines externen Lösungsmittels durchgeführt.

Falls die Veresterung in Gegenwart eines externen Lösungsmittels durchgeführt wird, handelt es sich dabei vorzugsweise um ein unter den Reaktionsbedingungen inertes organisches Lösungsmittel. Dazu gehören beispielsweise aliphatische Kohlenwasserstoffe, halogenierte aliphatische Kohlenwasserstoffe, aromatische und substituierte aromatische Kohlenwasserstoffe oder Ether. Bevorzugt ist das Lösungsmittel ausgewählt unter Pentan, Hexan, Heptan, Ligroin, Petrolether, Cyclohexan, Dichlormethan, Trichlormethan, Tetrachlormethan, Benzol, Toluol, Xylol, Chlorbenzol, Dichlorbenzolen, Dibutylether, THF, Dioxan, N,N-Dimethylformamid, Dimethylsulfoxid, Polyether, beispielsweise Polyalkylenglycole, wie Polyethylenglycole (PEG), Polyalkylenglycolmono-(C₁-C₄)-alkylether, wie Polyethylenglycolmonomethylether (MPEG), Polyalkylenglycol-di-(C₁-C₄)-alkylether, wie Polyethylenglycoldimethylether, mit einem Siedepunkt von über 200°C, und Mischungen davon.

In der Regel beträgt der Anteil der ionischen Flüssigkeit in der Reaktionsmischung 1 bis 50 Gew.-%, bevorzugt 2 bis 45 Gew.-%, insbesondere 5 bis 40 Gew.-%, bezogen auf das Gesamtgewicht der Reaktionsmischung.

Nach Beendigung der Reaktion besteht das im Sumpf befindliche Reaktionsgemisch üblicherweise aus zwei Phasen, einer organischen Phase, welche den gewünschten Ester und überschüssigem Alkohol enthält, und einer ionischen Fraktion, welche die ionische Flüssigkeit, den Katalysator und/oder dessen Folgeprodukte sowie geringe Mengen an Estercarbonsäure(n) und/oder nicht umgesetzter Carbonsäure enthält.

In der Regel wird nach beendeter Reaktion die ionische Fraktion, die den wenigstens einen Katalysator sowie gegebenenfalls nicht umgesetzte Carbonsäure und/oder nicht umgesetztes Carbonsäureanhydrid enthält, vom Reaktionsgemisch abtrennt und für weitere Veresterungsreaktionen wiederverwendet.

Üblicherweise erfolgt die Auftrennung des Reaktionsgemisches in die organische Phase und die ionische Fraktion über einen Prozess der Selbstentmischung (Phasentrennung). Hierzu wird das Reaktionsgemisch in einen Phasenscheider (Dekanter) geleitet, wo es durch mechanisches Absetzen in zwei Phasen (der organischen Produktphase und der ionischen Fraktion) zerfällt, die getrennt abgezogen werden können.

Falls sich die Auftrennung nicht oder nur unvollständig auf dem Wege der Selbstentmischung (Phasentrennung) erreichen lässt, kann die Auftrennung zusätzlich oder stattdessen extraktiv erfolgen. Dabei wird die Reaktionsmischung in der Regel unter Verwendung eines Lösungsmittels, welches die Esterverbindung sehr gut löst aber sich mit der ionischen Flüssigkeit nur geringfügig oder gar nicht mischt, extrahiert. Alternativ kann zur extraktiven Auftrennung des Reaktionsgemisches auch ein Lösungsmittel verwendet werden, welches die ionische Flüssigkeit sehr gut löst aber sich mit der Esterverbindung nur geringfügig oder gar nicht mischt.

Zur Extraktion geeignete Lösungsmittel, welche sich mit der ionischen Flüssigkeit nur geringfügig oder gar nicht mischen, sind beispielsweise ausgewählt unter aliphatischen Kohlenwasserstoffen, wie Pentan, Hexan, Heptan, Ligroin, Petrolether Cyclopentan oder Cyclohexan, halogenierten aliphatischen Kohlenwasserstoffen, wie Dichlormethan, Trichlormethan, Tetrachlormethan, oder 1,2-Dichlorethan aromatischen Kohlenwasserstoffen, wie Benzol, Toluol, Xylol, halogenierten aromatischen Kohlenwasserstoffen, wie Chlorbenzol, Dichlorbenzolen, Ethern, wie Diethylether, Methyl-tert.-Butylether, Dibutylether, Tetrahydrofuran oder Dioxan, sowie C₁-C₄-Alkylnitrilen, wie Acetonitril oder Propionitril, und dergleichen.

Bevorzugt wird zur extraktiven Auftrennung des Reaktionsgemisches ein Lösungsmittel verwendet werden, welches die ionische Flüssigkeit sehr gut löst aber sich mit der Esterverbindung nur geringfügig oder gar nicht mischt. Hierfür geeignete Lösungsmittel sind beispielsweise ausgewählt unter Wasser und C₁-C₄-Alkohlen, wie Methanol, Ethanol oder 2-Propanol, insbesondere Wasser.

Nach erfolgter Abtrennung kann die ionische Fraktion, welche die ionische Flüssigkeit, den Katalysator und/oder dessen Folgeprodukte sowie geringe Mengen an Estercarbonsäure(n) und/oder nicht umgesetzter Carbonsäure enthält, aufbewahrt und für weitere Umsetzungen wiederverwendet werden.

Nach erfolgter Abtrennung kann die organischen Phase, welche den gewünschten Ester und überschüssigem Alkohol enthält, weiteren Aufarbeitungs- und/oder Reinigungsschritten unterzogen werden.

In der Regel wird die organische Phase zur weiteren Aufarbeitung mit wässriger Base gewaschen, um gegebenenfalls darin enthaltene saure Verbindungen zu neutralisieren und/oder zu entfernen. Anschließend wird der überschüssige Alkohol destillativ entfernt. Die Reihenfolge der Prozessschritte kann dabei variiert werden. Dabei wird der größte Teil des nicht umgesetzten Alkohols bei Normaldruck oder im Vakuum abdestilliert. Die letzten Spuren des Alkohols können z. B. durch Wasserdampfdestillation, insbesondere im Temperaturbereich von 120 bis 225 °C unter Vakuum, entfernt werden. Die Abtrennung des Alkohols kann als erster oder als letzter Aufarbeitungsschritt erfolgen.

Die Neutralisation der sauren Stoffe, wie Carbonsäuren, Estercarbonsäuren oder gegebenenfalls der sauren Katalysatoren, erfolgt durch Zugabe von Basen, z. B. von Alkali- und/oder Erdalkalimetallcarbonaten, -hydrogencarbonaten oder -hydroxiden. Das Neutralisationsmittel kann in fester Form oder bevorzugt als Lösung, insbesondere als wässrige Lösung eingesetzt werden. Hier wird häufig Natronlauge einer Konzentration von 0.1 bis 30 Gew.-%, bevorzugt von 0.5 bis 15 Gew.-%, insbesondere von 1 bis 10 Gew.-% verwendet. Das Neutralisationsmittel wird in einer Menge zugesetzt, die dem Einfachen bis dem Vierfachen, insbesondere dem Einfachen bis Zweifachen der stöchiometrisch notwendigen Menge, die durch Titration bestimmt wird, entspricht.

Bei der Destillation wird auch, soweit vorhanden, der als Nebenprodukt entstandene Ether des eingesetzten Alkohols R¹-OH entfernt. Dieser ist in den nach dem erfindungsgemäßen Verfahren erhaltenen Reaktionsgemischen im Allgemeinen in einer Menge von < 2 Gew.-%, bevorzugt < 1 Gew.-%, enthalten (bestimmt durch qualitative GC-Messungen an derivatisierten Proben). Der erhaltene Ether kann gewünschtenfalls durch saure Etherspaltung wieder in den Alkohol R¹-OH überführt werden.

Der überschüssige Alkohol R¹-OH kann entweder direkt wiederverwendet werden oder weiter gereinigt werden, z. B. mittels Destillation.

Der erhaltene Carbonsäureester ist im Wesentlichen frei von festen Verunreinigungen. Er kann jedoch einer Filtration unterzogen werden, um eventuell im Reaktor befindliche Schwebstoffe zu entfernen.

Erfindungsgemäß eingesetzte Alkohole R¹-OH sind C₅-C₁₃-Alkanole. Die C₅-C₁₃-Alkanole können geradkettig oder verzweigt sein oder aus Gemischen aus geradkettigen und verzweigten C₅-C₁₃-Alkanole bestehen. Zu den bevorzugten C₅-C₁₃-Alkanolen zählen beispielsweise n-Pentanol, 2-Methylbutanol, n-Hexanol, n-Heptanol, Isoheptanol, n-Octanol, Isooctanol, 2-Ethylhexanol, n-Nonanol, Isononanol, Isodecanol, 2-Propylheptanol, n-Undecanol, Isoundecanol, n-Dodecanol, Isododecanol, n-Tridecanol oder Isotridecanol sowie Gemische davon. Besonders bevorzugt sind C₇-C₁₂-Alkanole.

Als Alkohole R¹-OH besonders bevorzugte C₇-C₁₂-Alkanole können geradkettig oder verzweigt sein oder aus Gemischen aus geradkettigen und verzweigten C₇-C₁₂-Alkanolen bestehen. Zu den besonders bevorzugten C₇-C₁₂-Alkanole zählen beispielsweise n-Octanol, 2-Ethylhexanol, n-Nonanol, Isononanol, Isodecanol, 2-Propylheptanol, n-Undecanol, Isoundecanol oder n-Dodecanol sowie Gemische davon. Insbesondere wird in dem erfindungsgemäßen Verfahren als Alkohol 2-Ethylhexanol eingesetzt.

Besonders bevorzugte C₇-C₁₂-Alkanole werden im Folgenden genauer definiert.

### Heptanol

Die im erfindungsgemäßen Verfahren eingesetzten Heptanole können geradkettig oder verzweigt sein oder aus Gemischen aus geradkettigen und verzweigten Heptanolen bestehen. Bevorzugt werden Gemische aus verzweigten Heptanolen, auch als Isoheptanol bezeichnet, verwendet, die durch die Rhodium- oder vorzugsweise Kobaltkatalysierte Hydroformylierung von Dimerpropen, erhältlich z. B. nach dem Dimersol®-Verfahren, und anschließende Hydrierung der erhaltenen Isoheptanale zu einem Isoheptanol-Gemisch, hergestellt werden. Entsprechend seiner Herstellung besteht das so gewonnene Isoheptanol-Gemisch aus mehreren Isomeren. Im Wesentlichen geradkettige Heptanole können durch die Rhodium- oder vorzugsweise Kobaltkatalysierte Hydroformylierung von 1-Hexen und anschließende Hydrierung des erhaltenen n-Heptanals zu n-Heptanol erhalten werden. Die Hydroformylierung von 1-Hexen bzw. Dimerpropen kann nach an sich bekannten Verfahren erfolgen: Bei der Hydroformylierung mit homogen im Reaktionsmedium gelösten Rhodiumkatalysatoren können sowohl unkomplexierte Rhodiumcarbonyle, die in situ unter den Bedingungen der Hydroformylierungsreaktion im Hydroformylierungsreaktionsgemisch unter Einwirkung von Synthesegas z. B. aus Rhodiumsalzen gebildet werden, als auch komplexe Rhodiumcarbonylverbindungen, insbesondere Komplexe mit organischen Phosphinen, wie Triphenylphosphin, oder Organophosphiten, vorzugsweise chelatisierenden Biphosphiten, wie z. B. in US-A 5288918 beschrieben, als Katalysator verwendet werden. Bei der Kobalt-katalysierten Hydroformylierung dieser Olefine werden im Allgemeinen homogen im Reaktionsgemisch lösliche Kobaltcarbonyl-Verbindungen eingesetzt, die sich unter den Bedingungen der Hydroformylierungsreaktion unter Einwirkung von Synthesegas in situ aus Kobaltsalzen bilden. Wird die Kobalt-katalysierte Hydroformylierung in Gegenwart von Trialkyl- oder Triarylphosphinen ausgeführt, bilden sich als Hydroformylierungsprodukt direkt die gewünschten Heptanole, so dass keine weitere Hydrierung der Aldehydfunktion mehr benötigt wird.

Zur Kobalt-katalysierten Hydroformylierung des 1-Hexens bzw. der Hexenisomerengemische eignen sich beispielsweise die in Falbe, New Syntheses with Carbon Monoxide, Springer, Berlin, 1980 auf den Seiten 162 - 168, erläuterten industriell etablierten Verfahren, wie das Ruhrchemie-Verfahren, das BASF-Verfahren, das Kuhlmann-Verfahren oder das Shell-Verfahren. Während das Ruhrchemie-, BASF- und das Kuhlmann-Verfahren mit nicht ligandmodifizierten Kobaltcarbonyl-Verbindungen als Katalysatoren arbeiten und dabei Hexanal-Gemische erhalten, verwendet das Shell-Verfahren (DE-A 1593368) Phosphin- oder Phosphit-Ligand-modifizierte Kobaltcarbonyl-Verbindungen als Katalysator, die aufgrund ihrer zusätzlichen hohen Hydrieraktivität direkt zu den Hexanolgemischen führen. Vorteilhafte Ausgestaltungen zur Durchführung der Hydroformylierung mit nicht-ligandmodifizierten Kobaltcarbonylkomplexen werden in DE-A 2139630, DE-A 2244373, DE-A 2404855 und WO 01014297 detailliert beschrieben.

Zur Rhodium-katalysierten Hydroformylierung des 1-Hexens bzw. der Hexenisomerengemische kann das industriell etablierte Rhodium-Niederdruck-Hydroformylierungs-verfahren mit Triphenylphosphinligand-modifizierten Rhodiumcarbonylverbindungen angewandt werden, wie es Gegenstand von US-A 4148830 ist. Vorteilhaft können zur Rhodium-katalysierten Hydroformylierung langkettiger Olefine, wie der nach den vorstehend genannten Verfahren erhaltenen Hexenisomerengemische nicht-ligand-modifizierte Rhodiumcarbonylverbindungen als Katalysator dienen, wobei im Unterschied zum Niederdruckverfahren ein höherer Druck von 80 bis 400 bar einzustellen ist. Die Durchführung solcher Rhodium-Hochdruck-Hydroformylierungs-verfahren wird in z. B. EP-A 695734, EP-B 880494 und EP-B 1047655 beschrieben.

Die nach Hydroformylierung der Hexen-Isomerengemische erhaltenen Isoheptanalgemische werden auf an sich herkömmliche Weise zu Isoheptanolgemischen katalytisch hydriert. Bevorzugt werden hierzu heterogene Katalysatoren verwendet, die als katalytisch aktive Komponente Metalle und/oder Metalloxide der VI. bis VIII. sowie der I. Nebengruppe des Periodensystems der Elemente, insbesondere Chrom, Molybdän, Mangan, Rhenium, Eisen, Kobalt, Nickel und/oder Kupfer, gegebenenfalls abgeschieden auf einem Trägermaterial wie Al₂O₃, SiO₂ und/oder TiO₂, enthalten. Solche Katalysatoren werden z. B. in DE-A 3228881, DE-A 2628987 und DE-A 2445303 beschrieben. Besonders vorteilhaft wird die Hydrierung der Isoheptanale mit einem Überschuss an Wasserstoff von 1,5 bis 20 % über der stöchiometrisch zur Hydrierung der Isoheptanale benötigten Wasserstoffmenge, bei Temperaturen von 50 bis 200 °C und bei einem Wasserstoffdruck von 25 bis 350 bar durchgeführt und zur Vermeidung von Nebenreaktionen dem Hydrierzulauf gemäß DE-A 2628987 eine geringe Menge Wasser, vorteilhaft in Form einer wässrigen Lösung eines Alkalimetallhydroxids oder -carbonats entsprechend der Lehre von WO 01087809 zugefügt.

### Octanol

2-Ethylhexanol, das für lange Jahre der in den größten Mengen produzierte Weichmacheralkohol war, kann über die Aldolkondensation von n-Butyraldehyd zu 2-Ethylhexenal und dessen anschließende Hydrierung zu 2-Ethylhexanol gewonnen werden (s. Ullmann's Encyclopedia of Industrial Chemistry; 5. Auflage, Bd. A 10, S. 137 - 140, VCH Verlagsgesellschaft GmbH, Weinheim 1987).

Im Wesentlichen geradkettige Octanole können durch die Rhodium- oder vorzugsweise Kobalt-katalysierte Hydroformylierung von 1-Hepten und anschließende Hydrierung des erhaltenen n-Octanals zu n-Octanol erhalten werden. Das dazu benötigte 1-Hepten kann aus der Fischer-Tropsch-Synthese von Kohlenwasserstoffen gewonnen werden.

Bei dem Alkohol Isooctanol handelt es sich im Unterschied zu 2-Ethylhexanol oder n-Octanol, bedingt durch seine Herstellungsweise, nicht um eine einheitliche chemische Verbindung sondern um ein Isomerengemisch aus unterschiedlich verzweigten C₈-Alkoholen, beispielsweise aus 2,3-Dimethyl-1-hexanol, 3,5-Dimethyl-1-hexanol, 4,5-Dimethyl-1-hexanol, 3-Methyl-1-heptanol und 5-Methyl-1-heptanol, die je nach den angewandten Herstellungsbedingungen und -verfahren in unterschiedlichen Mengenverhältnissen im Isooctanol vorliegen können. Isooctanol wird üblicherweise durch die Codimerisierung von Propen mit Butenen, vorzugsweise n-Butenen, und anschließende Hydroformylierung des dabei erhaltenen Gemisches aus Heptenisomeren hergestellt. Das bei der Hydroformylierung erhaltene Octanal-Isomerengemisch kann anschließend auf an sich herkömmliche Weise zum Isooctanol hydriert werden.

Die Codimerisierung von Propen mit Butenen zu isomeren Heptenen kann vorteilhaft mit Hilfe des homogenkatalysierten Dimersol®-Verfahrens (Chauvin et al; Chem. Ind.; Mai 1974, S. 375 - 378) erfolgen, bei dem als Katalysator ein löslicher Nickel-Phosphin-Komplex in Gegenwart einer Ethylaluminiumchlor-Verbindung, beispielsweise Ethylaluminiumdichlorid, dient. Als Phosphin-Liganden für den NickelkomplexKatalysator können z. B. Tributylphosphin, Tri-isopropyl-phosphin, Tricyclohexylphosphin und/oder Tribenzylphosphin eingesetzt werden. Die Umsetzung findet bei Temperaturen von 0 bis 80°C statt, wobei vorteilhaft ein Druck eingestellt wird, bei dem die Olefine im flüssigen Reaktionsgemisch gelöst vorliegen (Cornils; Hermann: Applied Homogeneous Catalysis with Organometallic Compounds; 2. Auflage; Bd. 1; S. 254 - 259, Wiley-VCH, Weinheim 2002).

Alternativ zum mit homogen im Reaktionsmedium gelösten Nickelkatalysatoren betriebenen Dimersol®-Verfahren kann die Codimerisierung von Propen mit Butenen auch mit auf einem Träger abgeschiedenen, heterogenen NiO-Katalysatoren durchgeführt werden, wobei ähnliche Hepten-Isomerenverteilungen erhalten werden wie beim homogen katalysierten Verfahren. Solche Katalysatoren werden beispielsweise im sogenannten Octol®-Verfahren (Hydrocarbon Processing, Februar 1986, S. 31 - 33) verwendet, ein gut geeigneter spezifischer Nickel-Heterogenkatalysator zur Olefindimerisierung bzw. Codimerisierung ist z. B. in WO 9514647 offenbart.

Anstelle von Katalysatoren auf Basis von Nickel können auch Brønsted-acide heterogene Katalysatoren zur Codimerisierung von Propen mit Butenen verwendet werden, wobei in der Regel höher verzweigte Heptene als in den Nickel-katalysierten Verfahren erhalten werden. Beispiele von hierfür geeigneten Katalysatoren sind feste Phosphorsäure-Katalysatoren z. B. mit Phosphorsäure imprägnierte Kieselgur oder Diatomeenerde, wie sie vom PolyGas®-Verfahren zur Olefindi- bzw. Oligomerisierung benutzt werden (Chitnis et al; Hydrocarbon Engineering 10, Nr. 6 - Juni 2005). Zur Codimerisierung von Propen und Butenen zu Heptenen sehr gut geeignete Brønsted-acide Katalysatoren sind Zeolithe, deren sich das auf Basis des PolyGas®-Verfahrens weiterentwickelte EMOGAS®-Verfahren bedient.

Das 1-Hepten und die Hepten-Isomerengemische werden nach den vorstehend in Zusammenhang mit der Herstellung von n-Heptanal und Heptanal-Isomerengemische erläuterten bekannten Verfahren mittels Rhodium- oder Kobalt-katalysierter Hydroformylierung, vorzugsweise Kobalt-katalysierter Hydroformylierung, in n-Octanal bzw. Octanal-Isomerengemische überführt. Diese werden anschließend z. B. mittels eines der vorstehend in Zusammenhang mit der n-Heptanol- und Isoheptanol-Herstellung genannten Katalysatoren zu den entsprechenden Octanolen hydriert.

### Nonanol

Im Wesentlichen geradkettiges Nonanol kann durch die Rhodium- oder vorzugsweise Kobaltkatalysierte Hydroformylierung von 1-Octen und nachfolgende Hydrierung des dabei erhaltenen n-Nonanals erhalten werden. Das Ausgangsolefin 1-Octen kann beispielsweise über eine Ethylenoligomerisierung mittels einem homogen im Reaktionsmedium - 1,4-Butandiol - löslichen Nickelkomplexkatalysator mit z. B. Diphenylphosphinoessigsäure oder 2-Diphenylphosphinobenzoesäure als Liganden erhalten werden. Dieses Verfahren ist auch unter der Bezeichnung Shell Higher Olefins Process oder SHOP-Verfahren bekannt (s. Weisermel, Arpe: Industrielle Organische Chemie; 5. Auflage; S. 96; Wiley-VCH, Weinheim 1998).

Bei der im erfindungsgemäßen Verfahren eingesetzte Alkoholkomponente Isononanol handelt es sich nicht um eine einheitliche chemische Verbindung sondern um ein Gemisch aus unterschiedlich verzweigten, isomeren C₉-Alkoholen, die je nach der Art ihrer Herstellung, insbesondere auch der verwendeten Ausgangsstoffe, unterschiedliche Verzweigungsgrade haben können. Im Allgemeinen werden die Isononanole durch Dimerisierung von Butenen zu Isooctengemischen, anschließende Hydroformylierung der Isooctengemische und Hydrierung der dabei erhaltenen Isononanalgemische zu Isononanolgemischen hergestellt, wie in Ullmann's Encyclopedia of Industrial Chemistry, 5. Auflage, Bd. A1, S. 291 - 292, VCH Verlagsgesellschaft GmbH, Weinheim 1995, erläutert.

Als Ausgangsmaterial zur Herstellung der Isononanole können Isobuten, cis- und trans-2-Buten als auch 1-Buten oder Gemische dieser Butenisomere verwendet werden. Bei der vorwiegend mittels flüssiger, z. B. Schwefel- oder Phosphorsäure, oder fester, z. B. auf Kieselgur, SiO₂ oder Al₂O₃ als Trägermaterial aufgebrachter Phosphorsäure oder Zeolithe oder Brønsted-Säuren katalysierten Dimerisierung von reinem Isobuten, wird überwiegend das stark verzweigte 2,4,4-Trimethylpenten, auch als Diisobutylen bezeichnet, erhalten, das nach Hydroformylierung und Hydrierung des Aldehyds hochverzweigte Isononanole liefert.

Bevorzugt sind Isononanole mit einem geringeren Verzweigungsgrad. Solche gering verzweigten Isononanol-Gemische werden aus den linearen Butenen 1-Buten, cis- und/oder trans-2-Buten, die gegebenenfalls noch geringere Mengen an Isobuten enthalten können, über den vorstehend beschriebenen Weg der Butendimerisierung, Hydroformylierung des Isooctens und Hydrierung der erhaltenen Isononanal-Gemische hergestellt. Ein bevorzugter Rohstoff ist das sogenannte Raffinat II, aus dem C₄-Schnitt eines Crackers, beispielsweise eines Steamcrackers, das nach Eliminierung von Allenen, Acetylenen und Dienen, insbesondere 1,3-Butadien durch dessen Partialhydrierung zu linearen Butenen oder dessen Abtrennung durch Extraktivdestillation, beispielsweise mittels N-Methylpyrrolidon, und nachfolgende Brønsted-Säure katalysierter Entfernung des darin enthaltenen Isobutens durch dessen Umsetzung mit Methanol oder Isobutanol nach großtechnisch etablierten Verfahren unter Bildung des Kraftstoffadditivs Methyl-tert.-Butylether (MTBE) oder des zur Gewinnung von Rein-Isobuten dienenden Isobutyl-tert.-Butylether, gewonnen wird.

Raffinat II enthält neben 1-Buten und cis- und trans-2-Buten noch n- und iso-Butan und Restmengen von bis zu 5 Gew.-% an Isobuten.

Die Dimerisierung der linearen Butene oder des im Raffinat II enthaltenen Butengemischs kann mittels der gängigen, großtechnisch praktizierten Verfahren, wie sie vorstehend in Zusammenhang mit der Erzeugung von Isoheptengemischen erläutert wurden, beispielsweise mittels heterogener, Brønsted-acider Katalysatoren wie sie im PolyGas®- oder EMOGAS®-Verfahren eingesetzt werden, mittels des Dimersol®-Verfahrens unter Verwendung homogen im Reaktionsmedium gelöster Nickel-Komplex-Katalysatoren oder mittels heterogener, Nickel(II)oxid-haltiger Katalysatoren nach dem Octol®-Verfahren oder dem Verfahren gemäß WO 9514647 durchgeführt werden. Die dabei erhaltenen Isoocten-Gemische werden nach den vorstehend in Zusammenhang mit der Herstellung von Heptanal-Isomerengemische erläuterten bekannten Verfahren mittels Rhodium- oder Kobalt-katalysierter Hydroformylierung, vorzugsweise Kobalt-katalysierter Hydroformylierung, in Isononanal-Gemische überführt. Diese werden anschließend z. B. mittels eines der vorstehend in Zusammenhang mit der Isoheptanol-Herstellung genannten Katalysatoren zu den geeigneten Isononanolgemischen hydriert.

Die so hergestellten Isononanol-Isomerengemische können über ihren Isoindex charakterisiert werden, der aus dem Verzweigungsgrad der einzelnen, isomeren Isononanolkomponenten im Isononanolgemisch multipliziert mit deren prozentualen Anteil im Isononanolgemisch errechnet werden kann. So tragen z. B. n-Nonanol mit dem Wert 0, Methyloctanole (eine Verzweigung) mit dem Wert 1 und Dimethylheptanole (2 Verzweigungen) mit dem Wert 2 zum Isoindex eines Isononanolgemisches bei. Je höher die Linearität, desto niedriger ist der Isoindex des betreffenden Isononanolgemisches. Dementsprechend kann der Isoindex eines Isononanolgemisches durch gaschromatographische Auftrennung des Isononanolgemisches in seine einzelnen Isomere und damit einhergehender Quantifizierung von deren prozentualen Mengenanteil im Isononanolgemisch, bestimmt nach Standardmethoden der gaschromatographischen Analyse, ermittelt werden. Zwecks Erhöhung der Flüchtigkeit und Verbesserung der gaschromatographischen Auftrennung der isomeren Nonanole werden diese zweckmäßigerweise vor der gaschromatographischen Analyse mittels Standardmethoden, beispielsweise durch Umsetzung mit N-Methyl-N-trimethylsilyltrifluoracetamid, trimethylsilyliert. Um eine möglichst gute Trennung der einzelnen Komponenten bei der gaschromatographischen Analyse zu erzielen, werden vorzugsweise Kapillarsäulen mit Polydimethylsiloxan als stationärer Phase verwendet. Solche Kapillarsäulen sind im Handel erhältlich und es bedarf lediglich einiger weniger Routineversuche des Fachmanns, um aus dem vielfältigen Angebot des Handels ein optimal für diese Trennaufgabe geeignetes Fabrikat auszuwählen.

Bei den im erfindungsgemäßen Verfahren eingesetzten Isononanolen handelt es sich im Allgemeinen um Isononanole mit einem Isoindex von 0,8 bis 2, vorzugsweise von 1,0 bis 1,8 und besonders bevorzugt von 1,1 bis 1,5 verestert bzw. verethert, die nach den vorstehend genannten Verfahren hergestellt werden können.

Lediglich beispielhaft werden im Folgenden mögliche Zusammensetzungen von Isononanolgemischen angegeben, wie sie im erfindungsgemäßen Verfahren eingesetzt werden können, wobei anzumerken ist, dass die Anteile der im Einzelnen aufgeführten Isomeren im Isononanolgemisch abhängig von der Zusammensetzung des Ausgangsmaterials, beispielsweise Raffinat II, dessen Zusammensetzung an Butenen produktionsbedingt variieren kann, und von Schwankungen in den angewandten Produktionsbedingungen, beispielsweise dem Alter der benutzten Katalysatoren und daran anzupassenden Temperatur- und Druckbedingungen, variieren können.

Beispielsweise kann ein Isononanolgemisch, das durch Kobalt-katalysierte Hydroformylierung und anschließende Hydrierung aus einem unter Verwendung von Raffinat II als Rohstoff mittels des Katalysators und Verfahrens gemäß WO 9514647 erzeugten Isooctengemisch hergestellt wurde, folgende Zusammensetzung haben:
- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
- 0,38 bis 1,38 Gew.-%, bevorzugt 0,48 bis 1,28 Gew.-%, besonders bevorzugt 0,58 bis 1,18 Gew.-% 2,6-Dimethylheptanol;
- 2,78 bis 4,78 Gew.-%, bevorzugt 2,98 bis 4,58 Gew.-%, besonders bevorzugt 3,28 bis 4,28 Gew.-% 3,5-Dimethylheptanol;
- 6,30 bis 16,30 Gew.-%, bevorzugt 7,30 bis 15,30 Gew.-%, besonders bevorzugt 8,30 bis 14,30 Gew.-% 3,6-Dimethylheptanol;
- 5,74 bis 11,74 Gew.-%, bevorzugt 6,24 bis 11,24 Gew.-%, besonders bevorzugt 6,74 bis 10,74 Gew.-% 4,6-Dimethylheptanol;
- 1,64 bis 3,64 Gew.-%, bevorzugt 1,84 bis 3,44 Gew.-%, besonders bevorzugt 2,14 bis 3,14 Gew.-% 3,4,5-Trimethylhexanol;
- 1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew.-% 4-Methyloctanol;
- 0,98 bis 2,98 Gew.-%, bevorzugt 1,18 bis 2,78 Gew.-%, besonders bevorzugt 1,48 bis 2,48 Gew.-% 5-Methyloctanol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew.-% 6-Methyloctanol;
- 0,1 bis 3 Gew.-%, bevorzugt 0,2 bis 2 Gew.-%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen; mit der Maßgabe, dass die die Gesamtsumme der genannten Komponenten 100 Gew.-% ergibt.

Entsprechend den vorstehenden Ausführungen kann ein Isononanolgemisch, das durch Kobalt-katalysierte Hydroformylierung und anschließende Hydrierung unter Verwendung eines Ethylen-haltigen Butengemisches als Rohstoff mittels des PolyGas®- oder EMOGAS®-Verfahrens erzeugten Isooctengemisches hergestellt wurde, im Bereich der folgenden Zusammensetzungen, abhängig von der Rohstoffzusammensetzung und Schwankungen der angewandten Reaktionsbedingungen variieren:
- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.-% 2-Methyloctanol;
- 5,7 bis 11,7 Gew.-%, bevorzugt 6,3 bis 11,3 Gew.-%, besonders bevorzugt 6,7 bis 10,7 Gew.-% 3-Ethylheptanol;
- 1,9 bis 3,9 Gew.-%, bevorzugt 2,1 bis 3,7 Gew.-%, besonders bevorzugt 2,4 bis 3,4 Gew.-% 2-Ethylheptanol;
- 1,7 bis 3,7 Gew.-%, bevorzugt 1,9 bis 3,5 Gew.-%, besonders bevorzugt 2,2 bis 3,2 Gew.-% 2-Propylhexanol;
- 3,2 bis 9,2 Gew.-%, bevorzugt 3,7 bis 8,7 Gew.-%, besonders bevorzugt 4,2 bis 8,2 Gew.-% 3,5-Dimethylheptanol;
- 6,0 bis 16,0 Gew.-%, bevorzugt 7,0 bis 15,0 Gew.-%, besonders bevorzugt 8,0 bis 14,0 Gew.-% 2,5-Dimethylheptanol;
- 1,8 bis 3,8 Gew.-%, bevorzugt 2,0 bis 3,6 Gew.-%, besonders bevorzugt 2,3 bis 3,3 Gew.-% 2,3-Dimethylheptanol;
- 0,6 bis 2,6 Gew.-%, bevorzugt 0,8 bis 2,4 Gew.-%, besonders bevorzugt 1,1 bis 2,1 Gew.-% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.-% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
mit der Maßgabe, dass sich die Gesamtsumme der genannten Komponenten zu 100% Gew.-% ergibt.

### Decanol

Bei der im erfindungsgemäßen Verfahren eingesetzten Alkoholkomponente Isodecanol handelt es sich nicht um eine einheitliche chemische Verbindung sondern um ein komplexes Gemisch unterschiedlich verzweigter, isomerer Decanole.

Diese werden im Allgemeinen durch die Nickel- oder Brønsted-Säure-katalysierte Trimerisierung von Propylen, beispielsweise nach dem vorstehend erläuterten PolyGas®- oder dem EMOGAS®-Verfahren, nachfolgende Hydroformylierung des dabei erhaltenen Isononen-Isomerengemisches mittels homogener Rhodium- oder Kobaltcarbonyl-Katalysatoren, vorzugsweise mittels Kobaltcarbonyl-Katalysatoren und Hydrierung des entstandenen Isodecanal-Isomerengemisches z. B. mittels der vorstehend in Zusammenhang mit der Herstellung von C₇- bis C₉-Alkoholen genannten Katalysatoren und Verfahren (Ullmann's Encyclopedia of Industrial Chemistry; 5. Auflage, Bd. A1, S. 293, VCH Verlagsgesellschaft GmbH, Weinheim 1985) hergestellt. Das so produzierte Isodecanol ist im Allgemeinen stark verzweigt.

Bei dem im erfindungsgemäßen Verfahren eingesetzten 2-Propylheptanol kann es sich um reines 2-Propylheptanol handeln oder um Propylheptanol-Isomerengemische handeln, wie sie im Allgemeinen bei der industriellen Herstellung von 2-Propylheptanol gebildet werden und gemeinhin ebenfalls als 2-Propylheptanol bezeichnet werden.

Reines 2-Propylheptanol kann durch Aldolkondensation von n-Valeraldehyd und nachfolgende Hydrierung des dabei gebildeten 2-Propylheptenals, beispielsweise gemäß US-A 2921089, erhalten werden. Im Allgemeinen enthält kommerziell erhältliches 2-Propylheptanol neben der Hauptkomponente 2-Propylheptanol herstellungsbedingt eines oder mehrere der 2-Propylheptanol-Isomeren 2-Propyl-4-methylhexanol, 2-Propyl-5-methylhexanol, 2-Isopropyl-heptanol, 2-Isopropyl-4-methylhexanol, 2-Isopropyl-5-methylhexanol und/oder 2-Propyl-4,4-dimethylpentanol. Die Anwesenheit anderer Isomere des 2-Propylheptanols, beispielsweise 2-Ethyl-2,4-dimethylhexanol, 2-Ethyl-2-methyl-heptanol und/oder 2-Ethyl-2,5-dimethylhexanol im 2-Propylheptanol ist möglich, aufgrund der geringen Bildungsraten der aldehydischen Vorläufer dieser Isomere im Zuge der Aldolkondensation sind diese, wenn überhaupt, nur in Spurenmengen im 2-Propylheptanol enthalten und spielen für die Weichmachereigenschaften des aus solchen 2-Propyheptanol-Isomerengemischen hergestellten Verbindungen praktisch keine Rolle.

Als Ausgangsmaterial zur Herstellung von 2-Propylheptanol können verschiedenerlei Kohlenwasserstoffquellen benutzt werden, beispielsweise 1-Buten, 2-Buten, Raffinat I - ein aus dem C₄-Schnitt eines Crackers nach Abtrennung von Allenen, Acetylenen und Dienen erhaltenes Alkan/Alken-Gemisch, das neben 1- und 2-Buten noch erhebliche Mengen an Isobuten enthält - oder Raffinat II, das aus Raffinat I durch Abtrennung von Isobuten erhalten wird und als Olefinkomponenten außer 1- und 2-Buten nur noch geringe Anteile an Isobuten enthält. Selbstverständlich können auch Gemische aus Raffinat I und Raffinat II als Rohstoff zur 2-Propylheptanol-Herstellung verwendet werden. Diese Olefine oder Olefingemische können nach an sich herkömmlichen Methoden mit Kobalt- oder Rhodium-Katalysatoren hydroformyliert werden, wobei aus 1-Buten ein Gemisch aus n- und iso-Valeraldehyd - die Bezeichnung iso-Valeraldehyd bezeichnet die Verbindung 2-Methylbutanal - gebildet wird, dessen n/iso-Verhältnis je nach verwendetem Katalysator und Hydroformylierungsbedingungen in relativ weiten Grenzen variieren kann. Beispielsweise wird bei Verwendung eines mit Triphenylphosphin modifizierten homogenen Rhodium-Katalysators (Rh/TPP) aus 1-Buten n- und iso-Valeraldehyd in einem n/iso-Verhältnis von im Allgemeinen 10:1 bis 20:1 gebildet, wohingegen bei Verwendung von mit Phosphit-Liganden, beispielsweise gemäß US-A 5288918 oder WO 05028407, oder von mit Phosphoamidit-Liganden, beispielsweise gemäß WO 0283695, modifizierten Rhodium-Hydroformylierungskatalysatoren fast ausschließlich n-Valeraldehyd gebildet wird. Während das Rh/TPP-Katalysatorsystem 2-Buten bei der Hydroformylierung nur sehr langsam umsetzt, so dass der größte Teil des 2-Butens aus dem Hydroformylierungsgemisch wieder zurückgewonnen werden kann, gelingt die Hydroformylierung des 2-Butens mit den erwähnten Phosphit-Ligand- oder Phosphoramidit-Ligand-modifizierten Rhodium-Katalysatoren, wobei vorwiegend n-Valeraldehyd gebildet wird. Hingegen wird im olefinischen Rohstoff enthaltenes Isobuten, wenn auch mit unterschiedlicher Geschwindigkeit, von praktisch allen Katalysatorsystemen zu 3-Methylbutanal und je nach Katalysator in geringerem Umfang zu Pivalaldehyd hydroformyliert.

Die je nach verwendeten Ausgangsmaterialien und Katalysatoren erhaltenen C₅-Aldehyde, d. h. n-Valeraldehyd gegebenenfalls im Gemisch mit iso-Valeraldehyd, 3-Methylbutanal und/oder Pivalaldehyd, können vor der Aldolkondensation gewünschtenfalls vollständig oder teilweise destillativ in die Einzelkomponenten aufgetrennt werden, so dass auch hier eine Möglichkeit besteht, die Isomerenzusammensetzung der im erfindungsgemäßen Herstellungsverfahren eingesetzten C₁₀-Alkoholkomponente zu beeinflussen und zu steuern. Desgleichen ist es möglich, das C₅-Aldehydgemisch, wie es bei der Hydroformylierung gebildet wird, ohne die vorherige Abtrennung einzelner Isomere der Aldolkondensation zuzuführen. Bei der Aldolkondensation, die mittels eines basischen Katalysators, wie einer wässrigen Lösung von Natrium- oder Kaliumhydroxid, beispielsweise nach den in EP-A 366089, US-A 4426524 oder US-A 5434313 beschriebenen Verfahren durchgeführt werden kann, entsteht bei Einsatz von n-Valeraldehyd als einziges Kondensationsprodukt 2-Propylheptenal, wohingegen bei Einsatz eines Gemisches isomerer C₅-Aldehyde ein Isomerengemisch aus den Produkten der Homoaldolkondensation gleicher Aldehydmoleküle und der gekreuzten Aldolkondensation unterschiedlicher Valeraldehyd-Isomere geformt wird. Selbstverständlich kann die Aldolkondensation durch die gezielte Umsetzung einzelner Isomere so gesteuert werden, dass überwiegend oder vollständig ein einzelnes Aldolkondensationsisomer gebildet wird. Die betreffenden Aldolkondensationsprodukte können anschließend, üblicherweise nach vorausgegangener, vorzugsweise destillativer Abtrennung aus der Reaktionsmischung und gewünschtenfalls destillativer Reinigung, mit herkömmlichen Hydrierkatalysatoren, beispielsweise den vorstehend zur Hydrierung von Aldehyden genannten, zu den entsprechenden Alkoholen oder Alkoholgemischen hydriert werden.

Im Allgemeinen werden im erfindungsgemäßen Verfahren Gemische des 2-Propylheptanols mit den genannten Propylheptanol-Isomeren eingesetzt, in denen der Gehalt an 2-Propylheptanol mindestens 50 Gew.-%, vorzugsweise 60 bis 98 Gew.-% und besonders bevorzugt 80 bis 95 Gew.-%, insbesondere 85 bis 95 Gew.-% beträgt.

Geeignete Mischungen von 2-Propylheptanol mit den Propylheptanol-Isomeren umfassen beispielsweise solche aus 60 bis 98 Gew.-% 2-Propylheptanol, 1 bis 15 Gew.-% 2-Propyl-4-methyl-hexanol und 0,01 bis 20 Gew.-% 2-Propyl-5-methyl-hexanol und 0,01 bis 24 Gew.-% 2-Isopropylheptanol, wobei die Summe der Anteile der einzelnen Bestandteile 100 Gew.-% nicht überschreitet. Bevorzugt addieren sich die Anteile der einzelnen Bestandteile zu 100 Gew.-%.

Weitere geeignete Mischungen aus 2-Propylheptanol mit den Propylheptanol-Isomeren umfassen beispielsweise solche aus 75 bis 95 Gew.-% 2-Propylheptanol, 2 bis 15 Gew.-% 2-Propyl-4-methyl-hexanol, 1 bis 20 Gew.-% 2-Propyl-5-methyl-hexanol, 0,1 bis 4 Gew.-% 2-Isopropylheptanol, 0,1 bis 2 Gew.-% 2-Isopropyl-4-methylhexanol und 0,1 bis 2 Gew.-% 2-Isopropyl-5-methyl-hexanol, wobei die Summe der Anteile der einzelnen Bestandteile 100 Gew.-% nicht überschreitet. Bevorzugt addieren sich die Anteile der einzelnen Bestandteile zu 100 Gew.-%.

Bevorzugte Mischungen von 2-Propylheptanol mit den Propylheptanol-Isomeren umfassen solche mit 85 bis 95 Gew.-% 2-Propylheptanol, 5 bis 12 Gew.-% 2-Propyl-4-methyl-hexanol und 0,1 bis 2 Gew.-% 2-Propyl-5-methylhexanol und 0,01 bis 1 Gew.-% 2-Isopropylheptanol, wobei die Summe der Anteile der einzelnen Bestandteile 100 Gew.-% nicht überschreitet. Bevorzugt addieren sich die Anteile der einzelnen Bestandteile zu 100 Gew.-%.

Bei Verwendung der genannten 2-Propylheptanol-Isomerengemische anstelle von reinem 2-Propylheptanol entspricht die Isomerenzusammensetzung der Alkylestergruppen der Produkte praktisch der Zusammensetzung der zur Veresterung verwendeten Propylheptanol-Isomerengemische.

### Undecanol

Die im erfindungsgemäßen Verfahren eingesetzten Undecanole können geradkettig oder verzweigt sein oder aus Gemischen geradkettiger und verzweigter Undecanole zusammengesetzt sein. Bevorzugt werden Gemische aus verzweigten Undecanolen, auch als Isoundecanol bezeichnet, als Alkoholkomponente verwendet.

Im Wesentlichen geradkettiges Undecanol kann durch die Rhodium- oder vorzugsweise Kobaltkatalysierte Hydroformylierung von 1-Decen und nachfolgende Hydrierung des dabei erhaltenen n-Undecanals erhalten werden. Das Ausgangsolefin 1-Decen wird über das zuvor bei der Herstellung von 1-Octen erwähnte SHOP-Verfahren hergestellt.

Zur Herstellung verzweigten Isoundecanols kann das im SHOP-Verfahren erhaltene 1-Decen einer Skelettisomerisierung z. B. mittels acider zeolithischer Molekularsiebe, wie in WO 9823566 beschrieben, unterzogen werden, wobei sich Gemische aus isomeren Decenen bilden, deren Rhodium- oder vorzugsweise Kobalt-katalysierte Hydroformylierung und nachfolgende Hydrierung der erhaltenen Isoundecanal-Gemische zu dem zur Herstellung der erfindungsgemäßen Verbindungen verwendeten Isoundecanols führt. Die Hydroformylierung von 1-Decen oder Isodecen-Gemischen mittels Rhodium- oder Kobalt-Katalyse kann wie zuvor in Zusammenhang mit der Synthese von C₇- bis C₁₀-Alkoholen beschrieben erfolgen. Entsprechendes gilt für die Hydrierung von n-Undecanal oder Isoundecanal-Gemischen zu n-Undecanol bzw. Isoundecanol.

Nach destillativer Reinigung des Austrags der Hydrierung können die so erhaltenen C₇-bis C₁₁-Alkylalkohole bzw. deren Gemische, in dem erfindungsgemäßen Verfahren verwendet werden.

### Dodecanol

Im Wesentlichen geradkettiges Dodecanol kann in vorteilhafter Weise über das Alfol®- oder Epal®-Verfahren gewonnen werden. Diese Verfahren beinhalten die Oxidation und Hydrolyse geradkettiger Trialkylaluminium-Verbindungen, welche ausgehend von Triethylaluminium schrittweise über mehrere Ethylierungsreaktionen unter Verwendung von Ziegler-Natta-Katalysatoren aufgebaut werden. Aus den daraus resultierenden Gemischen weitgehend geradkettiger Alkylalkohole unterschiedlicher Kettenlänge kann nach dem destillativen Austrag der C₁₂-Alkylalkohol-Fraktion das gewünschte n-Dodecanol erhalten werden.

Alternativ kann n-Dodecanol auch durch Hydrogenierung natürlicher Fettsäuremethylester, beispielsweise aus Kokosnussöl, hergestellt werden.

Verzweigtes Isododecanol kann analog zu den zuvor beschriebenen Verfahren zur Codimerisierung und/oder Oligomerisierung von Olefinen mit nachfolgender Hydroformylierung und Hydrierung der Isoundecen-Gemische erhalten werden. Nach destillativer Reinigung des Austrags der Hydrierung können die so erhaltenen Isododecanole bzw. deren Gemische, wie vorstehend beschrieben, in dem erfindungsgemäßen Verfahren verwendet werden.

Die in dem erfindungsgemäßen Verfahren eingesetzten Carbonsäuren sind ausgewählt unter aromatischen Mono-, Di-, Tri- oder Tetracarbonsäuren, aliphatischen Mono- und Dicarbonsäuren, Hydroxycarbonsäuren, alicyclischen Mono-, Di-, Tri- und Tetracarbonsäuren, heterocyclischen Dicarbonsäuren, den Anhydriden der zuvor genannten Carbonsäuren und Mischungen davon.

Bei den im erfindungsgemäßen Verfahren eingesetzten aromatischen Mono-, Di-, Tri- oder Tetracarbonsäuren und deren Anhydride handelt es sich beispielsweise um Benzoesäure, Benzoesäureanhydrid, Phthalsäure, Phthalsäureanhydrid, Isophthalsäure, Terephthalsäure, Trimellitsäure, Trimellitsäureanhydrid, Pyromellitsäure und Pyromellitsäuredianhydrid.

Bei den im erfindungsgemäßen Verfahren eingesetzten aliphatischen Mono- und Dicarbonsäuren handelt es sich beispielsweise um gesättigte Mono- und Dicarbonsäuren wie Essigsäure, Buttersäure, Valeriansäure, Bernsteinsäure, Adipinsäure oder Sebacinsäure, um ungesättigte Mono- und Dicarbonsäuren wie Acrylsäure, Maleinsäure oder der Fumarsäure sowie gegebenenfalls um die Anhydride der zuvor genannten Carbonsäuren.

Bei den im erfindungsgemäßen Verfahren eingesetzten Hydroxycarbonsäuren handelt es sich beispielsweise um Glycolsäure, Milchsäure, Äpfelsäure, Weinsäure oder Citronensäure sowie gegebenenfalls um deren Anhydride.

Bei den im erfindungsgemäßen Verfahren eingesetzten alicyclischen Mono-, Di-, Tri- und Tetracarbonsäuren handelt es sich beispielsweise um die kernhydrierten Derivate der zuvor genannten aromatischen Mono-, Di-, Tri- oder Tetracarbonsäuren, wie Cyclohexancarbonsäure, 1,2-Cyclohexandicarbonsäure, 1,3-Cyclohexandicarbonsäure, 1,4-Cyclohexandicarbonsäure, 1,2,4-Cyclohexantricarbonsäure oder 1,2,4,5-Cyclohexantetracarbonsäure sowie gegebenenfalls um deren Anhydride.

Bei den im erfindungsgemäßen Verfahren eingesetzten heterocyclischen Dicarbonsäuren handelt es sich beispielsweise um 2,5-Furandicarbonsäure oder 2,5-Tetrahydrofurandicarbonsäure.

In einer bevorzugten Ausführungsform des erfindungsgemäßen Verfahrens ist die Carbonsäure ausgewählt unter Benzoesäure, Benzoesäureanhydrid, Phthalsäure, Phthalsäureanhydrid, Isophthalsäure, Terephthalsäure, Trimellitsäure, Trimellitsäureanhydrid, Pyromellitsäure und Pyromellitsäuredianhydrid. Besonders bevorzugt ist die Carbonsäure ausgewählt unter Benzoesäure, Benzoesäureanhydrid, Terephthalsäure, Trimellitsäure, Trimellitsäureanhydrid. Insbesondere wird in dem erfindungsgemäßen Verfahren als Carbonsäure Terephthalsäure eingesetzt.

### Weichmacheranwendung

In Kunststoffen, bei denen die optischen Eigenschaften im Vordergrund stehen, ist es in der Regel wünschenswert, dass die zu deren Herstellung eingesetzten Weichmacher eine geringe Eigenfärbung, d. h. eine niedrige Farbzahl, aufweisen.

Die mit dem erfindungsgemäßen Verfahren hergestellten Carbonsäureester zeichnen sich insbesondere durch eine niedrige Farbzahl aus. Sie eignen sich somit in vorteilhafter Weise für den Einsatz als Weichmacher oder in Weichmachern für thermoplastische Polymere und Elastomere.

Darüberhinaus weisen die mit dem erfindungsgemäßen Verfahren hergestellten Carbonsäureester, bedingt durch den Einsatz hochreiner Methansulphonsäure (Lutropur® MSA oder Lutropur® MSA 100) als Katalysator, einen geringen Gesamtchlorgehalt als auch einen geringen Sulfatgehalt auf.

Die mit dem erfindungsgemäßen Verfahren hergestellten Carbonsäureester lassen sich in der Regel in allen thermoplastisch verarbeitbaren Polymeren einsetzen, zu deren Herstellung Weichmacher zum Einsatz kommen. Bevorzugt sind diese thermoplastischen Polymere ausgewählt unter Polyvinylchlorid (PVC), Polyvinylbutyral (PVB), Homo- und Copolymere von Vinylacetat, Homo- und Copolymere von Styrol, Polyacrylate, thermoplastische Polyurethane (TPU) oder Polysulfide und Mischungen davon.

Die mit dem erfindungsgemäßen Verfahren hergestellten Carbonsäureester können zudem bei der Herstellung von Elastomeren eingesetzt werden. Bevorzugt handelt es sich dabei um natürlichen Kautschuk (NR) oder auf synthetischem Wege hergestellte Kautschuke, wie beispielsweise Polyisopren-Kautschuk (IR), Styrol-Butadien-Kautschuk (SBR), Butadien-Kautschuk (BR), Nitril-Butadien-Kautschuk (NBR) oder Chloropren-Kautschuk (CR).

Die Erfindung wird anhand der im Folgenden beschriebenen Beispiele näher erläutert. Dabei sollen die Beispiele nicht als einschränkend für die Erfindung verstanden werden.

In den nachfolgenden Beispielen bzw. den Figuren werden folgende Abkürzungen verwendet:
MSA steht für Methansulfonsäure,
PTSA steht für para-Toluolsulfonsäure,
Basionics ST 35 steht für 1-Ethyl-3-methylimidazoliummethansulfonat,
MSTFA steht für N-Methyl-N-(trimethylsilyl)trifluoroacetamid,
DOTP steht für Bis(2-ethylhexyl)terephthalat (Dioctylterephthalat),
APHA steht für American Public Health Association,

### BEISPIELE

### I) Analytische Untersuchungen:

### I.a Gaschromatographische Untersuchung:

Für gaschromatographische Untersuchungen wurden die Proben mit einem Überschuss MSTFA (N-Methyl-N-(trimethylsilyl) trifluoroacetamid) versetzt und für 30 min auf 100 °C erhitzt, sodass alle aciden Protonen in die entsprechenden Trimethylsilylgruppen überführt waren. Nach dem Abkühlen wurden die Proben mit N,N-Dimethylformamid (DMF) verdünnt.

Angaben zum gaschromatographisches Trennsystem und zur Trennmethode:
Messgerät: Agilent 6890 Series
Injektor: Split/Splitless mit Split Liner siltec-deactivated (Restec # 20713-214.5)
Säule: Optima 1 (Länge= 25 m, Innendurchmesser = 0,25 mm, Außendurchmesser = 0,40 mm, Filmdicke 0,25 µm) der Firma Macherey & Nagel
Detektor: FID mit 300 ml/min Luft, 30 ml/min Wasserstoff und 30 ml/min Make-Up-Gas (Stickstoff)
Trägergas: Stickstoff
Fluss: 0,7 ml/min bei 8,3 PSI (bei einer Ofentemperatur von 80 °C)
Split: 1:36, Splitflow: 28 ml/min, Septum purge 2,0 ml/min (bei einer Ofentemperatur von 80° C)
Injektortemperatur: 340 °C
Injektionsvolumen: 1 µl
Detektortemperatur: 320 °C
Temperaturprogramm:
   Start:120 °C
   Verweildauer 1:0 min
   Temperaturrampe 1:20 °C/min
   Endtemperatur 1:350 °C
   Verweildauer 2:5 min
   Gesamtlaufzeit: 16,5 min

Wenn die Proben Hochsieder enthalten, kann die Verweildauer 2 alternativ auf 30 min gestellt werden. Die Gesamtlaufzeit erhöht sich dann auf 41,5 min.
Auswertung: Empower-3-Software nach Flächen-%
Retentionszeiten:
   DOTP (Peak 1) 10,456 min (Hauptpeak)
   DOTP (Peak 2) 10,202 min (Isomer von Peak 1)
   2-Ethyl-1-Hexanol-MSTFA 2,87 min
   2-Ethyl-1-Hexylmesylat 4,44 min
   Terephthalsäure-MSTFA 6,39 min
   Monoester-MSTFA 8,52 min
   Ethylhexanol-di-Ether 4,89 min

### I.b Bestimmung der Säurezahl:

Die Bestimmung der Säurezahl, angegeben in mg KOH / g Probe, erfolgt in Propanol durch potentiometrische Titration mit 0,1 mol / L Tetrabutylammoniumhydroxid-Maßlösung. Die Bestimmung wird auf Geräte und Elektroden der Firma Metrohm durchgeführt.

### I.c Bestimmung der Hazen-Farbzahl nach APHA:

Die Messung der Hazen-Farbzahl erfolgt in Anlehnung an die DIN/EN/ISO 6271-2 (März 2005) in Substanz gegen Wasser als Referenz. Zur Messung werden Rundküvetten mit einem Durchmesser von 11 mm verwendet. Als Messgerät kann z. B. ein Photometer Dr. Lange LICO 400 eingesetzt werden.

### II) Herstellungsbeispiele:

### Beispiel 1:

Synthese von DOTP aus Terephthalsäure und 2-Ethylhexanol mit MSA als Katalysator und 1-Ethyl-3-methylimidazoliummethansulfonat (Basionics ST 35) als Cosolvens.

In einem 1.6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Terephthalsäure (249 g, 1,50 mol), 2-Ethylhexanol (469 g, 3,60 mol) und Methansulfonsäure (Lutropur MSA, BASF, 6,13 g einer ca. 70 Gew.-% wässrigen Lösung, 0,045 mol) und 1-Ethyl-3-methylimidazolium-methansulfonat (Basionics ST 35, 55 g) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt, wobei sich ein azeotropes Gemisch aus Wasser und 2-Ethylhexanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung der Reaktion verwendet. Um einen konstanten Destillatstrom zu gewährleisten wurde die Temperatur innerhalb von 3 h stufenweise auf 200 °C und schließlich innerhalb von 1.5 h auf 224 °C erhöht. Danach lagen beide Phasen des Reaktionsgemisches als klare Lösungen vor. Nach einer Reaktionszeit von 4.5 h betrug der Umsatz bezogen auf die ausgekreiste Menge Wasser 90 %. Zusätzlich wurde das Reaktionsgemisch gaschromatographisch untersucht. Dabei wurde überraschenderweise gefunden, dass < 1 % der eingesetzten Menge 2-Ethylhexanol durch Nebenreaktionen verloren gegangen war. Nach dem Abkühlen auf 80°C wurden die Phasen getrennt und die ionische Fraktion (63 g), die neben der ionischen Flüssigkeit auch den Katalysator und die nicht umgesetzte Terephthalsäure enthielt, abgetrennt und zum Recycling aufbewahrt. Die Phase, die DOTP und 2-Ethylhexanol enthält wurde mit 1,8 % NaOH (25 g) und Wasser (200 mL) gewaschen und anschließend der Überschuss 2-Ethylhexanol sowie alle weiteren Verbindungen mit einem Siedepunkt unterhalb des Siedepunktes von DOTP im Vakuum abgezogen (220 °C, 5 mbar). Das so erhaltene Produkt wurde über eine Druckfilternutsche filtriert. Reaktionszeit: 4.5 h. Ausbeute: 85 %. GC-Gehalt: 98.77 % DOTP (Flächen%). Farbzahl (APAH, Hazen): 30.

### Beispiel 2:

Synthese von DOTP aus Terephthalsäure und 2-Ethylhexanol mit MSA als Katalysator und der ionischen Phase aus Beispiel 1.

In einem 1.6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Terephthalsäure (249 g, 1.50 mol), 2-Ethylhexanol (469 g, 3.60 mol) und die ionische Fraktion aus Beispiel 1 (55 g) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt, wobei sich ein azeotropes Gemisch aus Wasser und 2-Ethylhexanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung des Reaktionsfortschritts verwendet. Um einen konstanten Destillatstrom zu gewährleisten wurde die Temperatur innerhalb von 2 h stufenweise auf 200 °C und schließlich innerhalb von 6 h auf 217 °C erhöht. Danach lagen beide Phasen des Reaktionsgemisches als klare Lösungen vor. Nach einer Reaktionszeit betrug von 8 h betrug der Umsatz bezogen auf die ausgekreiste Menge Wasser 90 %. Zusätzlich wurde das Reaktionsgemisch gaschromatographisch untersucht. Dabei wurde überraschenderweise gefunden, dass nur 0.56 % der ursprünglich eingesetzten Menge 2-Ethylhexanol zu den entsprechenden Olefinen zersetzt wurde. Die Verluste durch Bildung weiterer Nebenprodukte (Mesylat, Ether) beliefen sich auf < 0.5 % der eingesetzten Menge 2-Ethylhexanol. Nach dem Abkühlen auf 80°C wurden die Phasen getrennt und die ionische Fraktion, die neben der ionischen Flüssigkeit auch den Katalysator und die nicht umgesetzte Terephthalsäure enthielt, abgetrennt und zum Recycling aufbewahrt. Die Phase, die DOTP und 2-Ethylhexanol enthält wurde mit 1.8 % NaOH (35 g) und Wasser (300 mL) gewaschen und anschließend überschüssiger Alkohol abdestilliert (220 °C, 8 mbar). Das so erhaltene Produkt wurde über eine Druckfilternutsche filtriert. Reaktionszeit: 8 h. Ausbeute: 90 %. HPLC-Gehalt: 99.64 % DOTP (Flächen%). Farbzahl (APAH, Hazen): 85, Säurezahl: 0.4 mgKOH/g.

### Vergleichsbeispiel V3:

Synthese von DOTP aus Terephthalsäure und 2-Ethylhexanol mit MSA als Katalysator ohne ionische Flüssigkeit.

In einem 1.6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Terephthalsäure (249 g, 1.50 mol), 2-Ethylhexanol (469 g, 3.60 mol) und Methansulfonsäure (Lutropur MSA, BASF, 6.13 g einer ca. 70 Gew% wässrigen Lösung, 0.045 mol) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt, wobei sich ein azeotropes Gemisch aus Wasser und 2-Ethylhexanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung der Reaktion verwendet. Um einen konstanten Destillatstrom zu gewährleisten und die Reaktion zu vollem Umsatz zu führen wurde die Temperatur innerhalb von 4 h stufenweise auf 200 °C und schließlich innerhalb von 2 h auf 215 °C erhöht. Dort wurde die Temperatur so lange gehalten, bis das Reaktionsgemisch als klare Lösung vorlag und die berechnete Menge Wasser aus der Reaktion und der eingesetzten Methansulfonsäure gesammelt war (56 g). Die Reaktionszeit betrug 7.5 h. Das Reaktionsgemisch wurde gaschromatographisch untersucht. Die Verluste durch Bildung von Nebenprodukten beliefen sich auf ca. 5 % der eingesetzten Menge 2-Ethylhexanol.

### Vergleichsbeispiel V4:

Synthese von DOTP aus Terephthalsäure und 2-Ethylhexanol mit Tetraisopropyltitanat als Katalysator und 1-Ethyl-3-methylimidazoliummethansulfonat (Basionics ST 35) als Cosolvens.

In einem 1.6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Terephthalsäure (249 g, 1.50 mol), 2-Ethylhexanol (469 g, 3.60 mol), Tetraisopropyltitanat (3.22 mmol) und 1-Ethyl-3-methylimidazolium-methansulfonat (Basionics ST 35, 36 g) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt, wobei sich ein azeotropes Gemisch aus Wasser und 2-Ethylhexanol bildete, das im Kondensator verflüssigt und in den Wasserabscheider überführt wurde. Nach der Phasentrennung wurde die organische Phase in den Reaktor zurückgeführt, während die wässrige Phase verworfen wurde. Die ausgeschiedene Menge Wasser wurde gewogen und zur Überwachung des Reaktionsfortschritts verwendet. Um einen konstanten Destillatstrom zu gewährleisten wurde die Temperatur innerhalb von 2 h stufenweise auf 200 °C und schließlich innerhalb von 6 h auf 219 °C erhöht. Die Reaktion zeigte nach 13 h keinen vollständigen Umsatz. Die beiden Phasen konnten nicht sauber getrennt werden und der Katalysator ließ sich nicht wiederverwenden. Der Ansatz wurde verworfen.

### Vergleichsbeispiel V5:

Synthese von DOTP aus Terephthalsäure und 2-Ethylhexanol mit MSA als Katalysator und Butylpyridiniumchlorid als ionische Flüssigkeit.

In einem 1.6 L Doppelmantel-Rührkesselreaktor, der über einen programmierbaren Thermostat geheizt werden kann und mit Ankerrührer, Wasserabscheider in Jennewein-Bauart, Kondensator, Stickstoffeinlass und Anschluss für eine Vakuumpumpe ausgestattet ist, wurden Terephthalsäure (249 g, 1.50 mol), 2-Ethylhexanol (469 g, 3.60 mol), Methansulfonsäure (Lutropur MSA, BASF, 6.13 g einer ca. 70 Gew% wässrigen Lösung, 0.045 mol) und Butylpyridiniumchlorid (36 g, 0.21 mol) vorgelegt und der Reaktor mit Stickstoff inertisiert. Der Stickstoffstrom durch die Apparatur wurde auf 2 - 4 L h⁻¹ eingestellt und das Reaktionsgemisch auf 180 °C erhitzt. Nachdem sich innerhalb von 5 h neben dem in der Methansulfonsäure-Lösung enthaltenen Wasser kein weiteres Wasser abgeschieden hatte, wurde der Ansatz verworfen.

### Vergleichsbeispiel V6:

Synthese von DOTP aus Terephthalsäure und 2-Ethylhexanol mit Diemethylzinndichlorid als Katalysator und Butylpyridiniumchlorid als ionische Flüssigkeit.

Die Versuchsdurchführung erfolgte analog zu Vergleichsbeispiel 5, wobei jedoch anstelle der Methansulfonsäure Dimethylzinndichlorid (0.57 g, 2.57 mmol) als Katalysator verwendet wurde. Nachdem sich innerhalb von 4 h kein Wasser abgeschieden hatte, wurde der Ansatz verworfen.

## Patentansprüche

1. Verfahren zur Herstellung von Carbonsäureestern, durch Umsetzung wenigstens einer Carbonsäure und/oder wenigstens eines Carbonsäureanhydrids, ausgewählt unter aromatischen Mono-, Di-, Tri- oder Tetracarbonsäuren, aliphatischen Mono- und Dicarbonsäuren, Hydroxycarbonsäuren, alicyclischen Mono-, Di-, Tri- und Tetracarbonsäuren, heterocyclischen Dicarbonsäuren, den Anhydriden der zuvor genannten Carbonsäuren und Mischungen davon, und wenigstens eines Alkohols R¹-OH, worin R¹ ausgewählt ist unter unverzweigten und verzweigten gesättigten C₅-C₁₃-Alkylresten, mit der Maßgabe, dass die Umsetzung
- in Gegenwart wenigstens einer ionischen Flüssigkeit, die ausgewählt ist unter Salzen der allgemeinen Formel (la), 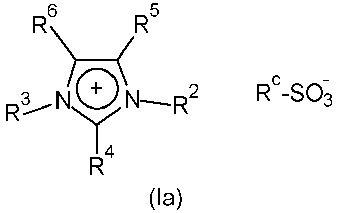 worin
Reste R² und R³ unabhängig voneinander für Wasserstoff, unsubstituiertes C₁-C₆-Alkyl, unsubstituiertes C₂-C₆-Alkenyl, unsubstituiertes C₅-C₆-Cycloalkyl, C₆-C₁₀-Aryl oder Heteroaryl mit 5 bis 6 Ringatomen, stehen, wobei die 2 letztgenannten Reste auch 1, 2 oder 3 Substituenten, ausgewählt unter C₁-C₆-Alkyl, C₁-C₆-Alkoxy oder Halogen, aufweisen können,
Reste R⁴, R⁵, und R⁶ unabhängig voneinander für Wasserstoff, unsubstituiertes C₁-C₆-Alkyl, unsubstituiertes C₅-C₆-Cycloalkyl oder Benzyl stehen, wobei Benzyl auch 1, 2 oder 3 Substituenten, ausgewählt unter C₁-C₆-Alkyl, C₁-C₆-Alkoxy oder Halogen, aufweisen kann,
und
R^{c} für unsubstituiertes C₁-C₆-Alkyl, unsubstituiertes C₅-C₆-Cycloalkyl oder Aryl steht, wobei Aryl mit 1, 2, oder 3 C₁-C₆-Alkylresten substituiert sein kann,
- in Gegenwart wenigstens eines Katalysators, der ausgewählt ist unter organischen Sulfonsäuren und
- unter destillativer Abtrennung wenigstens eines Teils des während der Reaktion gebildeten Wassers in Form einer azeotropen Mischung mit dem eingesetzten Alkohol R¹-OH,
erfolgt, wobei der abdestillierte Alkohol R¹-OH zumindest teilweise in das Reaktionssystem zurückgeführt wird.

2. Verfahren nach Anspruch 1, wobei die Umsetzung unter Zuführung eines unter den Reaktionsbedingungen inerten Gases zum Reaktionssystem erfolgt.

3. Verfahren nach einem der vorhergehenden Ansprüche, wobei das Anion der wenigstens einen ionischen Flüssigkeit der deprotonierten Form der als Katalysator eingesetzten organischen Sulfonsäure entspricht.

4. Verfahren nach einem der Ansprüche 2 oder 3, wobei die Umsetzung wenigstens einer Carbonsäure und/oder wenigstens eines Carbonsäureanhydrids und wenigstens eines Alkohols R¹-OH in wenigstens einem Reaktor durchgeführt wird, wobei man das inerte Gas in den wenigstens einen Reaktor unterhalb der Flüssigkeitsoberfläche einführt und das Reaktionsgemisch von dem inerten Gas durchperlt wird.

5. Verfahren nach einem der vorhergehenden Ansprüche, wobei die Umsetzung in einer Kaskade aus wenigstens zwei Reaktoren erfolgt.

6. Verfahren nach Anspruch 5, wobei das inerte Gas zumindest dem ersten Reaktor der Kaskade zugeführt wird.

7. Verfahren nach einem der vorhergehenden Ansprüche, wobei die Umsetzung in kontinuierlicher Fahrweise durchgeführt wird.

8. Verfahren nach einem der vorangegangenen Ansprüche, wobei der Rest R¹ ausgewählt ist unter n-Octyl, 2-Ethylhexyl, n-Nonyl, Isononyl, Isodecyl, 2-Propylheptyl, n-Undecyl, Isoundecyl.

9. Verfahren nach einem der vorangegangenen Ansprüche, wobei die Carbonsäure ausgewählt ist unter Benzoesäure, Benzoesäureanhydrid, Phthalsäure, Phthalsäureanhydrid, Isophthalsäure, Terephthalsäure, Trimellitsäure, Trimellitsäureanhydrid, Pyromellitsäure und Pyromellitsäuredianhydrid.

10. Verfahren nach einem der vorangegangenen Ansprüche zur Herstellung von Bis(2-ethylhexyl)terephthalat durch Umsetzung von Terephthalsäure mit 2-Ethylhexanol.

11. Verfahren nach einem der vorangegangenen Ansprüche, wobei der wenigstens eine Alkohol R¹-OH in einem 1,01- bis 2,0-fachen molaren Überschuss, bezogen auf die Carbonsäureäquivalente des eingesetzten Carbonsäurematerials, vorliegt.

12. Verfahren nach einem der vorangegangenen Ansprüche, wobei der Katalysator wenigstens eine Alkylsulfonsäure umfasst oder aus wenigstens einer Alkylsulfonsäure besteht.

13. Verfahren nach einem der vorangegangenen Ansprüche, wobei als Katalysator Methansulfonsäure eingesetzt wird.

14. Verfahren nach einem der vorangegangenen Ansprüche, wobei der Katalysator in einer Menge von 0,5 bis 5 mol%, bezogen auf die Anzahl der umzusetzenden Carbonsäuregruppen, eingesetzt wird.

15. Verfahren nach einem der vorangegangenen Ansprüche, wobei man nach beendeter Reaktion eine ionische Fraktion, die die ionische Flüssigkeit, den wenigstens einen Katalysator sowie gegebenenfalls nicht umgesetzte Carbonsäure und/oder nicht umgesetztes Carbonsäureanhydrid enthält, vom Reaktionsgemisch abtrennt und für weitere Veresterungsreaktionen wiederverwendet.

## Claims

1. A process for the production of carboxylic esters by reaction of at least one carboxylic acid and/or at least one carboxylic anhydride, selected from aromatic mono-, di-, tri-, or tetracarboxylic acids, aliphatic mono- and dicarboxylic acids, hydroxycarboxylic acids, alicyclic mono-, di-, tri-, and tetracarboxylic acids, heterocyclic dicarboxylic acids, the anhydrides of the abovementioned carboxylic acids, and mixtures thereof, and at least one alcohol R¹-OH, in which R¹ is selected among unbranched and branched saturated C₅-C₁₃-alkyl moieties, with the proviso that the reaction takes place
- in the presence of at least one ionic liquid which is selected from among salts of the general formula (Ia), 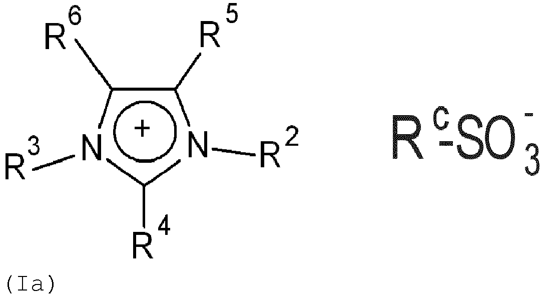 in which
radicals R² and R³, independently of one another, are hydrogen, unsubstituted C₁-C₆-alkyl, unsubstituted C₂-C₆-alkenyl, unsubstituted C₅-C₆-cycloalkyl, C₆-C₁₀-aryl or heteroaryl having 5 to 6 ring atoms, it also being possible for the 2 last-mentioned radicals to have 1, 2 or 3 substituents selected from C₁-C₆-alkyl, C₁-C₆-alkoxy or halogen,
radicals R⁴, R⁵, and R⁶, independently of one another, are hydrogen, unsubstituted C₁-C₆-alkyl, unsubstituted C₅-C₆-cycloalkyl or benzyl, it also being possible for benzyl to have 1, 2 or 3 substituents selected from C₁-C₆-alkyl, C₁-C₆-alkoxy or halogen,
and
R^{c} is unsubstituted C₁-C₆-alkyl, unsubstituted C₅-C₆-cycloalkyl or aryl, where aryl may be substituted by 1, 2 or 3 C₁-C₆-alkyl radicals,
- in the presence of at least one catalyst selected among organic sulfonic acids, and
- with distillative removal, in the form of an azeotropic mixture with the alcohol R¹-OH used, of at least one portion of the water formed during the reaction,
where at least to some extent the alcohol R¹-OH removed by distillation is returned to the reaction system.

2. The process according to claim 1, where the reaction takes place with supply to the reaction system of a gas which is inert under the reaction conditions.

3. The process according to any of the preceding claims, where the anion of the at least one ionic liquid corresponds to the deprotonated form of the organic sulfonic acid used as catalyst.

4. The process according to either of claims 2 and 3, where the reaction of at least one carboxylic acid and/or of at least one carboxylic anhydride and of at least one alcohol R¹-OH is carried out in at least one reactor, where the inert gas is passed into the at least one reactor below the liquid surface, and the inert gas is bubbled through the reaction mixture.

5. The process according to any of the preceding claims, where the reaction takes place in a cascade made of at least two reactors.

6. The process according to claim 5, where the inert gas is introduced at least into the first reactor of the cascade.

7. The process according to any of the preceding claims, where the reaction is carried out continuously.

8. The process according to any of the preceding claims, where the radical R¹ is selected from n-octyl, 2-ethylhexyl, n-nonyl, isononyl, isodecyl, 2-propylheptyl, n-undecyl, isoundecyl.

9. The process according to any of the preceding claims, where the carboxylic acid is selected from benzoic acid, benzoic anhydride, phthalic acid, phthalic anhydride, isophthalic acid, terephthalic acid, trimellitic acid, trimellitic anhydride, pyromellitic acid, and pyromellitic dianhydride.

10. The process according to any of the preceding claims for the production of bis(2-ethylhexyl) terephthalate by reaction of terephthalic acid with 2-ethylhexanol.

11. The process according to any of the preceding claims, where the at least one alcohol R¹-OH is present in a 1.01- to 2.0-fold molar excess, based on the carboxylic acid equivalents of the carboxylic acid material used.

12. The process according to any of the preceding claims, where the catalyst comprises at least one alkylsulfonic acid or consists of at least one alkylsulfonic acid.

13. The process according to any of the preceding claims, where methanesulfonic acid is used as catalyst.

14. The process according to any of the preceding claims, where the amount used of the catalyst, based on the number of the carboxylic acid groups to be reacted, is from 0.5 to 5 mol%.

15. The process according to any of the preceding claims, where after the reaction has ended, an ionic fraction which comprises the ionic liquid, the at least one catalyst and also any unreacted carboxylic acid and/or unreacted carboxylic anhydride is removed from the reaction mixture and used again for further esterification reactions.

## Revendications

1. Procédé de fabrication d'esters d'acides carboxyliques, par mise en réaction d'au moins un acide carboxylique et/ou d'au moins un anhydride d'acide carboxylique, choisi parmi les acides mono-, di-, tri- ou tétracarboxyliques aromatiques, les acides mono- et dicarboxyliques aliphatiques, les acides hydroxycarboxyliques, les acides mono-, di-, tri- et tétracarboxyliques alicycliques, les acides dicarboxyliques hétérocycliques, les anhydrides des acides carboxyliques mentionnés précédemment et leurs mélanges, et d'au moins un alcool R¹-OH, R¹ étant choisi parmi les radicaux alkyle en C₅-C₁₃ saturés non ramifiés ou ramifiés, à condition que la réaction ait lieu
- en présence d'au moins un liquide ionique, qui est choisi parmi les sels de formule générale (Ia) 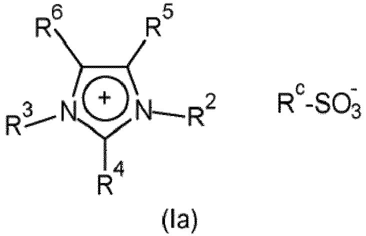 dans laquelle
les radicaux R² et R³ représentent indépendamment l'un de l'autre l'hydrogène, un alkyle en C₁-C₆ non substitué, un alcényle en C₂-C₆ non substitué, un cycloalkyle en C₅-C₆ non substitué, un aryle en C₆-C₁₀ ou un hétéroaryle de 5 à 6 atomes de cycle, les 2 derniers radicaux cités pouvant également comprendre 1, 2 ou 3 substituants choisis parmi alkyle en C₁-C₆, alcoxy en C₁-C₆ ou halogène, les radicaux R⁴, R⁵ et R⁶ représentent indépendamment les uns des autres l'hydrogène, un alkyle en C₁-C₆ non substitué, un cycloalkyle en C₅-C₆ non substitué ou un benzyle, le benzyle pouvant également comprendre 1, 2 ou 3 substituants, choisis parmi alkyle en C₁-C₆, alcoxy en C₁-C₆ ou halogène,
et
R^{c} représente un alkyle en C₁-C₆ non substitué, un cycloalkyle en C₅-C₆ non substitué ou un aryle, l'aryle pouvant être substitué avec 1, 2 ou 3 radicaux alkyle en C₁-C₆,
- en présence d'au moins un catalyseur, qui est choisi parmi les acides sulfoniques organiques, et
- avec séparation par distillation d'au moins une partie de l'eau formée pendant la réaction sous la forme d'un mélange azéotropique avec l'alcool R¹-OH utilisé, l'alcool R¹-OH éliminé par distillation étant au moins partiellement recyclé dans le système réactionnel.

2. Procédé selon la revendication 1, dans lequel la réaction a lieu avec introduction d'un gaz inerte dans les conditions de réaction dans le système réactionnel.

3. Procédé selon l'une quelconque des revendications précédentes, dans lequel l'anion dudit au moins un liquide ionique correspond à la forme déprotonée de l'acide sulfonique organique utilisé en tant que catalyseur.

4. Procédé selon l'une quelconque des revendications 2 ou 3, dans lequel la réaction d'au moins un acide carboxylique et/ou d'au moins un anhydride d'acide carboxylique et d'au moins un alcool R¹-OH est réalisée dans au moins un réacteur, le gaz inerte étant introduit dans ledit au moins un réacteur en dessous de la surface du liquide et le gaz inerte barbotant dans le mélange réactionnel.

5. Procédé selon l'une quelconque des revendications précédentes, dans lequel la réaction a lieu dans une cascade d'au moins deux réacteurs.

6. Procédé selon la revendication 5, dans lequel le gaz inerte est introduit au moins dans le premier réacteur de la cascade.

7. Procédé selon l'une quelconque des revendications précédentes, dans lequel la réaction est réalisée en mode continu.

8. Procédé selon l'une quelconque des revendications précédentes, dans lequel le radical R¹ est choisi parmi n-octyle, 2-éthylhexyle, n-nonyle, isononyle, isodécyle, 2-propylheptyle, n-undécyle, isoundécyle.

9. Procédé selon l'une quelconque des revendications précédentes, dans lequel l'acide carboxylique est choisi parmi l'acide benzoïque, l'anhydride de l'acide benzoïque, l'acide phtalique, l'anhydride de l'acide phtalique, l'acide isophtalique, l'acide téréphtalique, l'acide triméllitique, l'anhydride de l'acide triméllitique, l'acide pyroméllitique et le dianhydride de l'acide pyroméllitique.

10. Procédé selon l'une quelconque des revendications précédentes, pour la fabrication de téréphtalate de bis(2-éthylhexyle) par mise en réaction d'acide téréphtalique avec du 2-éthylhexanol.

11. Procédé selon l'une quelconque des revendications précédentes, dans lequel ledit au moins un alcool R¹-OH est présent en un excès molaire d'un facteur de 1,01 à 2,0, par rapport aux équivalents d'acide carboxylique du matériau acide carboxylique utilisé.

12. Procédé selon l'une quelconque des revendications précédentes, dans lequel le catalyseur comprend au moins un acide alkylsulfonique ou est constitué par au moins un acide alkylsulfonique.

13. Procédé selon l'une quelconque des revendications précédentes, dans lequel de l'acide méthanesulfonique est utilisé en tant que catalyseur.

14. Procédé selon l'une quelconque des revendications précédentes, dans lequel le catalyseur est utilisé en une quantité de 0,5 à 5 % en moles, par rapport au nombre de groupes acide carboxylique à mettre en réaction.

15. Procédé selon l'une quelconque des revendications précédentes, dans lequel une fraction ionique, qui contient le liquide ionique, ledit au moins un catalyseur, ainsi qu'éventuellement l'acide carboxylique non réagi et/ou l'anhydride d'acide carboxylique non réagi, est séparée du mélange réactionnel après la fin de la réaction, et réutilisée pour d'autres réactions d'estérification.
